# EUROPÄISCHE PATENTSCHRIFT

(11) **EP 0 923 508 B1**
(45) Veröffentlichungstag und Bekanntmachung des Hinweises auf die Patenterteilung: **14.11.2001**
(21) Anmeldenummer: 97937556.5
(22) Anmeldetag: 06.08.1997
(51) Int. Cl.: C07B 39/00

(54) **VERFAHREN ZUR HERSTELLUNG VON FLUOR ENTHALTENDEN VERBINDUNGEN**
PROCESS FOR PREPARING FLUORINE-CONTAINING COMPOUNDS
PROCEDE DE PREPARATION DE COMPOSES FLUORES

(30) Priorität: 07.08.1996 DE 19631854
(43) Veröffentlichungstag der Anmeldung: 23.06.1999
(73) Patentinhaber: Aventis Research & Technologies GmbH & Co. KG, 65926 Frankfurt am Main (DE)
(72) Erfinder: KOLOMEITSEV, Alexander, Kiev-133, 252133 (UA); PASENOK, Sergej, D-65779 Kelkheim (DE)
(74) Vertreter: Brundin, Eike, Dr.
(86) Internationale Anmeldenummer: EP9704284
(87) Internationale Veröffentlichungsnummer: WO9805610

(56) Entgegenhaltungen:
- EP-A- 0 556 737
- WO-A-92/00270
- GB-A- 2 042 507

## Beschreibung

Die vorliegende Erfindung betrifft ein gegenüber dem Stand der Technik verbessertes Verfahren zur Herstellung von Fluor enthaltenden Verbindungen mittels einer Halogen-Fluor-Austauschreaktion.

Fluor enthaltende Verbindungen finden unter anderem in flüssigkristallinen Mischungen Anwendung (EP 0 602 596).

Die Halogen-Fluor-Austauschreaktion ist auch unter dem Namen Halex-Reaktion bekannt. Sie stellt eine häufig praktizierte Methode dar, Fluor-Substituenten in eine Verbindung, die gegen Fluor austauschbares Halogen enthält, einzuführen.

Bei aromatischen Verbindungen, insbesondere bei aktivierten aromatischen Verbindungen, verläuft der Halogen-Fluor-Austausch im Sinne einer nucleophilen Substitution. Zur Durchführung dieser Reaktion werden vergleichweise hohe Reaktionstemperaturen benötigt, die häufig zwischen 200 und 300°C liegen, wodurch zum Teil erhebliche Anteile an Zersetzungsprodukten entstehen. Im allgemeinen kann auf ein Lösungsmittel nicht verzichtet werden, so daß die Raum/Zeitausbeuten im Vergleich zu lösungsmittelfreien Verfahren deutlich niedriger liegen. Als Alternative hierzu können herkömmliche Phasentransfer-Katalysatoren verwendet werden, durch die sich einige der vorstehend genannten Nachteile verringern lassen.

Andere Probleme, wie eine schlechte Rührbarkeit der Reaktionssuspension bei lösungsmittelfreien Verfahren, bleiben weiter bestehen. Bislang wurden als Phasentransfer-Katalysatoren quartäre Alkylammonium- oder Alkylphosphoniumsalze (US-PS 4287374), Pyridiniumsalze (WO 87/04194), Kronenether oder Tetraphenylphosphoniumsalze (J.H. Clark et al., Tetrahedron Letters 28 [1987], Seiten 111 bis 114) verwendet. Diese Phasentransfer-katalysatoren weisen zum Teil vergleichsweise geringe Aktivität auf und sind unter den für die Durchführung der Reaktion erforderlichen Temperaturen nur mäßig stabil.

In Anbetracht dieser Einschränkungen und Nachteile besteht ein großes Bedürfnis nach einem Verfahren, das die den bekannten Verfahren innewohnenden Nachteile, insbesondere hohe Reaktionstemperaturen und lange Reaktionszeiten, vermeidet und zudem die gewünschten Fluor enthaltenden Verbindungen in guter bis sehr guter Ausbeute bei niedrigeren Reaktionstemperaturen und kürzeren Reaktionszeiten zugänglich macht.

Gelöst wird diese Aufgabe durch ein Verfahren zur Herstellung von Fluor enthaltenden Verbindungen durch Umsetzung einer Verbindung, die gegen Fluor austauschbares Halogen enthält, mit einem Fluorid oder einem Gemisch von Fluoriden der allgemeinen Formel I

MeF (I),

worin Me für ein Erdalkalimetallion, NH₄⁺-ion oder Alkalimetallion steht, in Anwesenheit oder Abweseheit eines Lösungsmittels bei einer Temperatur von 40 bis 260°C dadurch gekennzeichnet, daß man die Umsetzung in Anwesenheit einer Verbindung oder eines Gemisches von Verbindungen der allgemeinen Formel (II) 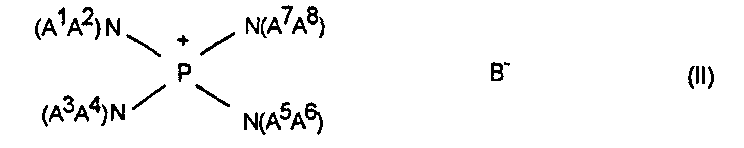
worin A¹, A², A³, A⁴, A⁵, A⁶, A⁷, A⁸ unabhängig voneinander, gleich oder verschieden sind und für ein geradkettiges oder verzweigtes Alkyl oder Alkenyl mit 1 bis 12 Kohlenstoffatomen, Cycloalkyl mit 4 bis 8 Kohlenstoffatomen, für ein Aryl mit 6 bis 12 Kohlenstoffatomen, ein Aralkyl mit 7 bis 12 Kohlenstoffatomen stehen, oder A¹ A², A³A⁴, A⁵A⁶, A⁷A⁸ unabhängig voneinander, gleich oder verschieden sind und direkt oder über O oder N-A⁹ miteinander zu einem Ring mit 3 bis 7 Ringgliedern verbunden sind, A⁹ für ein Alkyl mit 1 bis 4 Kohlenstoffatomen steht und B⁻ einen einwertigen Säurerest oder das Äquivalent eines mehrwertigen Säurerests bedeutet, durchführt.

Es ist als überraschend anzusehen, daß die Verwendung der Verbindungen der allgemeinen Formel (II) als Katalysator zu einer starken Beschleunigung der Reaktion führt, wodurch die Halogen-Fluor-Austauschreaktion (Halex-Reaktion) unter erheblich schonenderen Bedingungen, insbesondere niedrigeren Temperaturen und/oder kürzeren Reaktionszeiten durchgeführt werden kann. Dadurch kann zugleich auch die Bildung unerwünschter Nebenprodukte zurückgedrängt oder weitgehend vermieden werden.

Das erfindungsgemäße Verfahren weist noch weitere Vorteile auf. So sind die Verbindungen der allgemeinen Formel (II) nicht toxisch oder allenfalls nur gering toxisch und besitzen darüber hinaus eine thermische Belastbarkeit, die der dem Stand der Technik bekannten und häufig eingesetzten Katalysatoren überlegen ist. Verwendet man eine Verbindung der allgemeinen Formel (II), in der die Reste A¹ bis A⁸ jeweils für eine Methylgruppe stehen und setzt diese Verbindung über 10 Stunden einer Temperatur von nicht weniger als 230°C aus, so finden sich danach lediglich 0,2 % kohleartige Zersetzungsprodukte.

Unter dem Begriff gegen Fluor austauschbares Halogen wird Chlor, Brom oder Jod, insbesondere Chlor oder Brom, bevorzugt Chlor, das durch Fluorid im Sinne einer nucleophilen Substitution ausgetauscht werden kann, verstanden.

Ein weiterer Vorteil des erfindungsgemäßen Verfahrens besteht darin, daß man eine Vielzahl von Verbindungen als Ausgangsmaterial einsetzen kann.

So ist es möglich, als Verbindung, die gegen Fluor austauschbares Halogen enthält, eine am Kern einen gegen Fluor austauschbaren Chlor- oder Bromsubstituenten, insbesondere Chlorsubstituenten, besitzende aromatische Verbindung mit 0 bis 3 Stickstoffatomen im Kern, die gegebenenfalls wenigstens einen weiteren, eine nucleophile Substitution der aromatischen Verbindung begünstigenden Substituenten aufweist, einzusetzen.

Ohne Anspruch auf Vollständigkeit zu erheben, kommen als Ausgangsverbindungen für das erfindungsgemäße Verfahren aromatische Verbindungen vom Benzol-, Naphthalin-, Pyridin-, Anthracen-, Phenanthren-, Pyrimidin- und Pyrazin-Typ sowie vom Typ benzoanellierter Ringsysteme des Pyridins (Chinolin-, Isochinolin-, Acridin-, Acridon-Typ), des Pyrimidins, Pyrazins und Piperazins (Benzodiazine des Cinnolin-, Phtalazin-, Chinazolin-, Chinoxalin-, Phenazin-, Phenoxazin-Typs) und deren Derivate in Betracht, die gegebenfalls wenigstens einen weiteren, die nucleophile Substitution der aromatischen Verbindung begünstigenden Substituenten aufweisen. Dieser weitere, die nucleophile Substitution der aromatischen Verbindung begünstigende Substituent führt üblicherweise zu einer Aktivierung der aromatischen Verbindung, die dadurch einer Halogen-Fluor-Austauschreaktion leichter zugänglich wird.

Bei dem weiteren, die nucleophile Substitution an der aromatischen Verbindung begünstigenden Substituenten handelt es sich um -J- und -M-Substituenten, die die Elektronendichte respektive die Nucleophilie des Aromaten herabsetzen und dadurch eine elektrophile Substitution erschweren. Der Aromat wird dadurch jedoch gegenüber einer nucleophilen Substitution aktiviert. Die aktivierende Wirkung dieser Substituenten ist besonders groß, wenn sie in ortho- oder para-Stellung zu dem gegen Fluor auszutauschenden Halogen, insbesondere Chlor oder Brom, bevorzugt Chlor, stehen.

Ohne Anspruch auf Vollständigkeit zu erheben, seien als weitere, die nucleophile Substitution und damit die Halogen-Fluor-Austauschreaktion, insbesondere die Chlor-Fluor-Austauschreaktion begünstigende Substituenten F, Cl, Br, J, NO₂, NO, CF₃, CN, CHO, COF, COCl, SO₂F, SO₂Cl, OCF₃, SCF₃, SOCF₃, SO₂CF₃, COOR, CONRR', SO₂R, COR, OR oder ein Rest -CO-O-CO-, -CO-NR-CO-, der zwei ortho-Stellungen verknüpft, insbesondere F, Cl, NO₂, CF₃, CN, CHO, COCI, SO₂Cl, COOR, SO₂CF₃, CONRR', SO₂R, COR, bevorzugt F, Cl, NO₂, CF₃, CN, CHO, COCl, wobei R und R' jeweils unabhängig voneinander, gleich oder verschieden sind, und für H, ein geradkettiges oder verzweigtes Alkyl mit 1 bis 6, insbesondere 1 bis 4 Kohlenstoffatomen, ein Aryl mit 6 bis 12 kohlenstoffatomen oder Aralkyl mit 7 bis 12 Kohlenstoffatomen und die Alkyle und Aralkyle gegebenenfalls einfach bis dreifach halogensubstituiert, insbesondere fluoriert oder chloriert, sind, genannt.

Man kann eine am Kern einen gegen Fluor austauschbaren Chlor- oder Bromsubstituenten, insbesondere Chlorsubstituenten, besitzende aromatische Verbindung einsetzen, die wenigstens einen weiteren Substituenten aus der Reihe F, Cl, Br, J, NO₂, CF₃, CN, CHO, COF, COCI, SO₂F, SO₂Cl, OCF₃, SCF₃, SOCF₃, SO₂CF₃, COOR, CONRR', SO₂R, COR oder OR oder einen Rest -CÖ-O-CO-, -CO-NR-CO-, der zwei ortho-Stellungen verknüpft, aufweist, wobei R und R' jeweils unabhängig voneinander, gleich oder verschieden sind, und für H, ein geradkettiges oder verzweigtes Alkyl mit 1 bis 6 Kohlenstoffatomen, ein Aryl mit 6 bis 12 Kohlenstoffatomen oder Aralkyl mit 7 bis 12 Kohlenstoffatomen stehen und die Alkyle und Aralkyle gegebenenfalls einfach bis dreifach halogensubstituiert sind.

Die vorstehend erwähnten aromatischen Verbindungen können auch zusätzliche Substituenten enthalten, beispielsweise Alkylreste, Aminogruppen, Alkylaminogruppen, Hydroxygruppen oder Alkoxygruppen.

Man kann als Ausgangsmaterial eine am Kern einen gegen Fluor austauschbaren Chlor- oder Bromsubstituenten, insbesondere Chlorsubstituenten, besitzende aromatische Verbindung, die wenigstens ein gegen Fluor austauschbares Chlor oder Brom, insbesondere Chlor, als weiteren Substituenten und gegebenenfalls wenigstens einen weiteren Substituenten aus der Reihe F, NO₂, CF₃, CN, CHO, COF, COCl, SO₂F, SO₂Cl, OCF₃, SCF₃, SO₂CF₃, COOR, CONRR', SO₂R, COR, OR, -CO-O-CO- oder -CO-NR-CO- besitzt, einsetzen. Diese Ausgangsverbindungen besitzen demzufolge wenigstens zwei gegen Fluor austauschbare Halogensubstituenten, die unabhängig voneinander für Chlor oder Brom, insbesondere Chlor, stehen können. Diese Verbindungen sind üblicherweise einem einfachen oder zweifachen Halogen-Fluor-Austausch zugänglich, ohne daß sie einen weiteren Substituenten aus der vorstehend genannten Reihe besitzen müssen. Sie können aber auch einen weiteren, die nucleophile Substitution der aromatischen Verbindung begünstigenden Substituenten aus der Reihe der zuvor genannten Reste besitzen. Die Anwesenheit der Substituenten erhöht die Reaktivität der aromatischen Verbindung hinsichtlich der Halogen-Fluor-Austauschreaktion.

Mit gutem Erfolg kann man in das erfindungsgemäße Verfahren eine Verbindung der allgemeinen Formel (III) 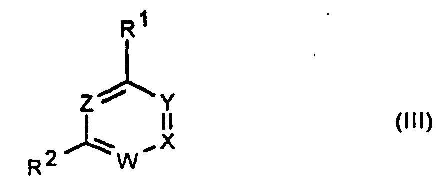 worin W für N oder C-R³, X für N oder C-R⁴, Y für N oder C-R⁵, Z für N oder C-R⁶ steht, W, X und Y nicht zugleich N sind, R¹, R², R³, R⁴, R⁵, R⁶ gleich oder verschieden sind und H, F, Cl, Br, J, NO₂, NO, CF₃, CN, CHO, COF, COCl, SO₂F, SO₂Cl, OCF₃, SCF₃, SO₂CF₃, COOR, CONRR', SO₂R, COR, OR, einen Rest -CO-O-CO-, -CO-NR-CO- oder -CR"=CR"-CR"=CR"-, der zwei ortho-Stellungen verknüpft, bedeuten, R und R' die vorstehende Bedeutung besitzen und R" unabhängig voneinander, gleich oder verschieden sind und dieselbe Bedeutung wie R¹ bis R⁶ haben, und wenigstens einer der Reste R¹ bis R⁶ Chlor oder Brom, insbesondere Chlor ist, einsetzen.

Man kann eine Verbindung der Formel (III), worin R¹, R², R³, R⁴, R⁵, R⁶ gleich oder verschieden sind und insbesondere H, F, Cl, Br, NO₂, CF₃, CN, CHO, COCl, bevorzugt H, F, Cl, NO₂, CN, CHO bedeuten, einsetzen.

Man kann auch eine Verbindung der Formel (III), worin nur einer der Reste R¹ bis R⁶ Chlor oder Brom, insbesondere Chlor, ist, keiner der Reste W, X, Y, Z für ein Stickstoffatom steht und wenigstens einer der verbleibenden Reste aus der Gruppe R¹ bis R⁶ NO₂, CF₃, CN, CHO, COF, COCl, SO₂F, SO₂Cl, OCF₃, SCF₃, SO₂CF₃, COOR, CONRR', SO₂R, COR, OR, -CO-O-CO-, -CO-NR-CO- oder -CR"=CR"-CR"=CR"- ist, einsetzen.

Man kann in das Verfahren eine Verbindung der Formel (III), worin 2 oder mehrere der Reste R¹ bis R⁶ Chlor oder Brom, insbesondere Chlor, sind, die Reste W, X, Y, Z für 0 bis 3 Stickstoffatome stehen und die verbleibenden Reste aus der Gruppe R¹ bis R⁶ alle Wasserstoff sein können, einsetzen.

Man kann in das Verfahren auch eine Verbindung der Formel (III), worin nur einer der Reste R¹ bis R⁶ Chlor oder Brom, insbesondere Chlor, ist, wenigstens einer der Reste W, X, Y, Z für ein Stickstoffatom steht und die verbleibenden Reste aus der Gruppe R¹ bis R⁶ alle Wasserstoff sein können, einsetzen.

Der Einbau wenigstens eines Stickstoffatomes in den aromatischen Ring erhöht die Reaktivität der aromatischen Verbindung derart, daß gegebenenfalls auch ohne Anwesenheit eines weiteren, die nucleophile Substitution der aromatischen Verbindung begünstigenden Substituenten ein Halogen-Fluor-Austausch stattfinden kann.

Mit gutem Erfolg kann man eine Verbindung der allgemeinen Formel (IV) 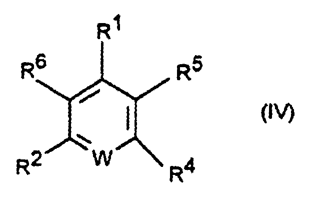 worin W für N oder C-R³ steht, einer der Reste R¹, R², R⁴, R⁵, R⁶ und gegebenenfalls R³Cl, F, NO₂, CF₃, CN, CHO, COF, COCl, SO₂F, SO₂Cl, OCF₃, SCF₃, SO₂CF₃, COOR, CONRR', SO₂R, COR oder OR ist oder zwei der Reste, die in ortho-Stellung zueinander stehen, -CO-O-CO- oder -CO-NR-CO- bedeuten, wobei R und R' jeweils unabhängig voneinander, gleich oder verschieden sind und für H, ein geradkettiges oder verzweigtes Alkyl mit 1 bis 6 Kohlenstoffatomen, ein Aryl mit 6 bis 12 Kohlenstoffatomen oder Aralkyl mit 7 bis 12 Kohlenstoffatomen stehen, ein weiterer der Reste R¹, R², R⁴, R⁵, R⁶ und gegebenenfalls R³ Cl ist und die übrigen Reste für H, F oder Cl stehen, einsetzen.

Mit guter Aussicht auf Erfolg kann man auch eine Verbindung der allgemeinen Formel (IV) 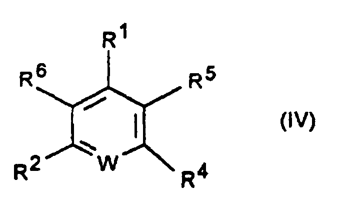 worin W für N oder C-R³ steht, einer der Reste R¹, R², R⁴, R⁵, R⁶ oder der Rest R³Cl, F, NO₂, CF₃, CN, CHO, COF, COCl, SO₂F, SO₂Cl, OCF₃, SCF₃, SO₂CF₃, COOR, CONRR', SO₂R, COR oder OR ist oder zwei der Reste, die in ortho-Stellung zueinander stehen, -CO-O-CO- oder -CO-NR-CO- bedeuten, wobei R und R' jeweils unabhängig voneinander, gleich oder verschieden sind und für H, ein geradkettiges oder verzweigtes Alkyl mit 1 bis 6 Kohlenstoffatomen, ein Aryl mit 6 bis 12 Kohlenstoffatomen oder Aralkyl mit 7 bis 12 Kohlenstoffatomen stehen, ein weiterer der Reste R¹, R², R⁴, R⁵, R⁶ Cl ist und die übrigen Reste für H, F oder Cl stehen, einsetzen.

Die Reste -CO-O-CO- und -CO-NR-CO- betreffen allgemein zwei der Reste R¹ bis R⁶, die in ortho-Stellung zueinander stehen, insbesondere zwei in ortho-Stellung zueinander stehende Reste aus der Gruppe R¹, R², R⁴, R⁵ und R⁶, falls W für N steht, oder zwei in ortho-Stellung zueinander stehende Reste aus der Gruppe R², R³ und R⁴, falls W für C-R³ steht.

In der Verbindung der Formel (IV) steht einer der Reste R¹, R², R⁴, R⁵, R⁶ und gegebenenfalls R³ oder der Rest R³ insbesondere für Cl, F, NO₂, CF₃, CN, CHO, COF, COCl, OCF₃, COOR, COONRR', COR, OR, -CO-O-CO- oder -CO-NR-CO-, bevorzugt für Cl, F, NO₂, CF₃, CN, CHO, COOR oder COCl, R und R' bedeuten insbesondere H, ein geradkettiges oder verzweigtes Alkyl mit 1 bis 4 Kohlenstoffatomen oder Aryl mit 6 bis 12 Kohlenstoffatomen, bevorzugt H oder ein geradkettiges oder verzweigtes Alkyl mit 1 bis 3 Kohlenstoffatomen, besonders bevorzugt Methyl oder Ethyl, einer oder zwei der Reste R¹, R², R⁴, R⁵, R⁶ und gegebenenfalls R³ stehen für Cl und die übrigen Reste sind gleich oder verschieden und stehen für H oder Cl.

Die vorstehend aufgeführte Formel (IV) umfaßt nicht aktivierte Verbindungen, in denen einer der Reste R¹, R², R⁴, R⁵, R⁶ und gegebenenfalls R³ für Cl oder F steht und zusätzlich einer, zwei oder mehrere der Reste R¹, R², R⁴, R⁵, R⁶ und gegebenenfalls R³ für Cl stehen und die daraus resultierenden Verbindungen ein, zwei oder mehr Cl enthalten, falls der eine der vorstehend genannten Reste F ist, oder zwei, drei oder mehr Cl enthalten, falls der eine der vorstehend genannten Reste nicht F sondern Cl ist.

Beispiele für derartige nichtaktivierte Derivate des Pyridins, wobei in Formel (IV) W für N steht, sind 2,3-Dichlorpyridin, 2,4-Dichlorpyridin, 2,5-Dichlorpyridin, 2,6-Dichlorpyridin, 3,4-Dichlorpyridin, 3,5-Dichlorpyridin, 2,3,4-Trichlorpyridin, 2,3,5-Trichlorpyridin, 2,3,6-Trichlorpyridin, 2,4,6-Trichlorpyridin, Tetrachlorpyridin und Pentachlorpyridin sowie fluorierte Chlorpyridine, die sich infolge Teilfluorierung aus den vorstehend genannten Chlorpyridinen bilden.

Beispiele für derartige nichtaktivierte Derivate des Benzols, wobei in Formel (IV) W für C-R³ steht, sind 1,2-Dichlorbenzol, 1,3-Dichlorbenzol, 1,4-Dichlorbenzol, 1,2,3-Trichlorbenzol, 1,2,4-Trichlorbenzol, 1,3,5-Trichlorbenzol, 1,2,3,4-Tetrachlorbenzol, 1,2,3,5-Tetrachlorbenzol, 1,2,4,5-Tetrachlorbenzol, aber auch fluorierte Chlorbenzole, die sich infolge Teilfluorierung aus den vorstehend genannten Chlorbenzolen bilden.
Die vorstehend aufgeführte Formel (IV) umfaßt auch Verbindungen, die einen aktivierenden Rest enthalten. Als aktivierender Rest kommen NO₂, CF₃, CN, CHO, COF, COCl, SO₂F, SO₂Cl, OCF₃, SCF₃, SO₂CF₃, COOR, COONRR', SO₂R, COR, OR, -CO-O-CO- oder -CO-NR-CO-, insbesondere NO₂, CF₃, CN, CHO, COF, COCI, OCF₃, COOR, CONRR', COR, OR, -CO-O-CO- oder -CO-NR-CO-, bevorzugt NO₂, CF₃, CN, CHO, COCI, COOR, COR in Betracht.

Bei den Verbindungen, die einen aktivierenden Rest enthalten, ist einer der Reste R¹ bis R⁶ in Formel (IV), insbesondere einer der Reste aus der Gruppe R¹, R², R⁴, R⁵, R⁶, falls W für N steht, oder insbesondere der Rest R³, falls W für C-R³ steht, der aktivierende Rest. Der aktivierende Rest entfaltet eine besonders große Wirkung, wenn das gegen F auszutauschende Cl in ortho- oder para-Stellung zu dem aktivierenden Rest steht. In diesem Zusammenhang sei nochmals erwähnt, daß das N-Atom im Pyridinring ebenfalls im Sinne eines Chlor-Fluor-Austausches aktivierend wirkt.

Das erfindungsgemäße Verfahren betrifft nicht nur den Austausch von Cl in ortho-Stellung und/oder para-Stellung zu einem aktivierenden Rest, sondern auch den Austausch von Cl in den weniger begünstigten meta-Stellungen. So kann man auch Verbindungen der allgemeinen Formel (V) 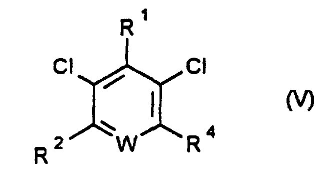 worin W für N oder C-R³ steht, wobei R³ NO₂, CF₃, CN, CHO, COF, COCI, SO₂F, SO₂Cl, OCF₃, SCF₃, SO₂CF₃, COOR, COONRR', SO₂R, COR, OR ist oder zwei in ortho-Stellung stehende Reste aus der Gruppe R², R³, R⁴ -CO-O-CO- oder -CO-NR-CO- sind, insbesondere NO₂, CF₃, CN, CHO, COF, COCl, OCF₃, COOR, CONRR', COR, OR ist oder zwei in ortho-Stellung stehende Reste aus der Gruppe R², R³, R⁴ sind, bevorzugt NO₂, CF₃, CN, CHO, COCI und R¹, R², R⁴ für H, F oder Cl stehen, einsetzen.

Die Formeln (III), (IV) und (V), die nachfolgend einander gegenübergestellt werden, 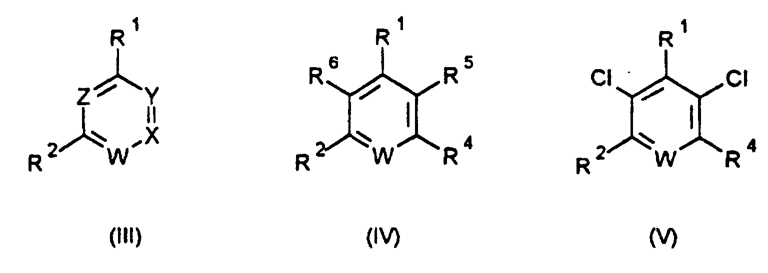 stehen zueinander in einem klaren Zusammenhang. Ersetzt man in Formel (III) X durch C-R⁴, Y durch C-R⁵ und Z durch C-R⁶, so gelangt man zu Formel (IV). Ersetzt man in Formel (IV) R⁵ und R⁶ durch Cl so erhält man Formel (V). Somit läßt sich auch die Formel (V) von Formel (III) ableiten. Auf diesen Zusammenhang sei an dieser Stelle aufmerksam gemacht, um mögliche Mißverständnisse zu vermeiden.

Ohne Anspruch auf Vollständigkeit zu erheben, seien folgende Stoffe, die gegen Fluor austauschbares Halogen enthalten, als kleine Auswahl genannt: 2-Chlornitrobenzol, 2,4-Dichlomitrobenzol, 2-Chlorbenzaldeyd, 4-Chlorbenzaldehyd, 2-Chlorbenzonitril, 4-Chlorbenzonitril, 2-Chlorbenzoylchlorid, 4-Chlorbenzoylchlorid, 2,4-Dichlorbenzaldehyd, 2,6-Dichlorbenzaldehyd, 2,4-Dichlorbenzonitril, 2,6-Dichlorbenzonitril, 2,4-Dichlorbenzoylchlorid und 2,6-Dichlorbenzoylchlorid.

Man setzt als Fluorid der Formel (I) Kalziumfluorid, Ammoniumfluorid, Lithiumfluorid, Natriumfluorid, Kaliumfluorid, Rubidiumfluorid, Cäsiumfluorid oder ein Gemisch derselben, insbesondere Lithiumfluorid, Natriumfluorid, Kaliumfluorid, Rubidiumfluorid, Cäsiumfluorid oder ein Gemisch derselben, bevorzugt Natriumfluorid, Kaliumfluorid, Cäsiumfluorid oder ein Gemisch derselben, besonders bevorzugt Kaliumfluorid, Cäsiumfluorid oder ein Gemisch derselben ein. Häufig genügt es, Kaliumfluorid allein einzusetzen.

Was das Mengenverhältnis Fluorid zu Ausgangsverbindungen anbelangt, so ist zu berücksichtigen, daß es Fälle geben kann, in denen ein Überschuß an Fluorid zu unerwünschten Nebenreaktionen führen kann. In diesen Fällen empfiehlt es sich, das Fluorid auch im Unterschuß einzusetzen. Man setzt üblicherweise das Fluorid der Formel (II):Äquivalent auszutauschendes Halogen im Verhältnis (0,5 bis 10):1, insbesondere (0,8 bis 5):1, bevorzugt (1 bis 2):1, besonders bevorzugt (1 bis 1,5):1 ein.

Wie eingangs bereits erwähnt, führt man die Umsetzung in Anwesenheit einer Verbindung der Formel (II), die als Katalysator fungiert, durch.

Die Verbindungen der Formel (II) lassen sich beispielsweise durch Umsetzung von Phosphorpentachlorid mit Dialkylaminen herstellen. Aus der nachfolgenden Gleichung ist die Umsetzung unter Verwendung von Dimethylamin zu entnehmen:

PCl₅ + HN(CH₃)₂ - P[N(CH₃)₂]₄ Cl

Man kann aber auch Phosphorpentachlorid stufenweise mit unterschiedlichen sekundären Aminen, beispielsweise Dialkylaminen, zur Reaktion bringen, um unsymmetrisch substituierte Verbindungen der Formel (II) zu erhalten. Weitere Möglichkeiten, Verbindungen der Formel (II) zu synthetisieren, sind von R. Schwesinger et al., Angew. Chem. 103 (1991) 1376 und R. Schwesinger et al., Chem. Ber. 127 (1994) 2435 bis 2454 beschrieben.

Man kann eine Verbindung der Formel (II), worin A¹, A², A³, A⁴, A⁵, A⁶, A⁷, A⁸ unabhängig voneinander, gleich oder verschieden sind und für ein geradkettiges oder verzweigtes Alkyl oder Alkenyl, insbesondere Alkyl, mit 1 bis 12, insbesondere 1 bis 8, bevorzugt 1 bis 4 Kohlenstoffatomen, oder Cycloalkyl mit 4 bis 8, insbesondere 5 bis 6 Kohlenstoffatomen, stehen, einsetzen. Diese Verbindungen sind von besonderem Interesse, da sie sich auf vergleichsweise einfache Art ausgehend von den entsprechenden Dialkylaminen, Dialkenylaminen, Dicycloalkylaminen, sekundären Aminen, die einen Alkyl- und Alkenylrest, einen Alkyl- und Cycloalkylrest oder einen Alkenyl- und Cycloalkylrest enthalten, herstellen lassen.

Als Beispiele für Alkyl sind Methyl, Ethyl, n-Propyl, i-Propyl, n-Butyl, i-Butyl, n-Pentyl, 3-Methylbutyl, n-Hexyl, 2-Ethylhexyl, insbesondere Methyl, Ethyl, n-Propyl, n-Butyl und als Beispiele für Alkenyl sind Allyl, Prop-(2)-enyl, n-But-(2)-enyl, und als Beispiele für Cycloalkyl sind Cyclopentyl, Cyclohexyl, 4-Methylcyclohexyl, 4-tert.-Butylcyclohexyl, zu nennen.

Man kann eine Verbindung der Formel (II), worin A¹A² = A³A⁴ oder A¹A² = A³A⁴ = A⁵A⁶ oder A¹A² = A³A⁴ = A⁵A⁶ = A⁷A⁸ ist, einsetzen. Diese Verbindungen, in denen zwei oder mehrere der Gruppen A¹A², A³A⁴, A⁵A⁶ und A⁷A⁸ einander gleich sind, sind relativ gut zugänglich.

Man kann auch eine Verbindung der Formel (II), worin A¹=A², A³=A⁴, A⁵=A⁶ und/oder A⁷=A⁸ ist, einsetzen. Diese Verbindungen sind vergleichsweise leicht zugänglich und deshalb von Interesse.

Man kann auch eine Verbindung der Formel (II), worin A¹ = A² = A³ = A⁴ oder A¹ = A² = A³ = A⁴ = A⁵ = A⁶ oder A¹ = A² = A³ = A⁴ = A⁵ = A⁶ = A⁷ = A⁸ ist, einsetzen. Diese vorstehend genannten Verbindungen, in denen vier, sechs oder acht der Reste A¹ bis A⁸ gleich sind, sind aufgrund ihrer leichten Zugänglichkeit ebenfalls von Interesse.

Es ist auch möglich, eine Verbindung der Formel (II), worin A¹A² oder A¹A² und A³ A⁴ oder A¹A² und A³A⁴ und A⁵A⁶ oder A¹A² und A³A⁴ und A⁵A⁶ und A⁷A⁸ direkt oder über O oder N-A⁹ miteinander zu einem gesättigten oder ungesättigten Ring mit 5 oder 6 Ringgliedern verbunden sind, einzusetzen. Dementsprechend enthalten diese Verbindungen einen, zwei, drei oder vier der vorstehend erwähnten Ringe.

Es ist ferner möglich, eine Verbindung der Formel (II), worin A¹A² oder A¹A² und A³ A⁴ oder A¹A² und A³A⁴ und A⁵A⁶ oder A¹A² und A³A⁴ und A⁵A⁶ und A⁷A⁸ zu einem Ring, der das N-Atom, an dem die jeweiligen Reste A¹ bis A⁸ sitzen, gegebenenfalls O oder N-A⁹ und CH₂-Gruppen als Ringglieder umfaßt, verbunden sind, einzusetzen. In dieser Stoffgruppe bilden das N-Atom mit den an ihnen jeweils befindlichen Resten A¹ bis A⁸ beispielsweise einen Hexahydropyridinring, Tetrahydropyrrolring, einen Hexahydropyrazinring oder Morpholinring. Dementsprechend enthalten diese Verbindungen einen, zwei, drei oder vier der vorstehend erwähnten Ringe.

In der Verbindung der Formel (II) steht B⁻, wie bereits eingangs erwähnt, für einen einwertigen Säurerest oder das Äquivalent eines mehrwertigen Säurerestes, insbesondere den Rest einer anorganischen Mineralsäure, einer organischen Carbonsäure, einer aliphatischen oder aromatischen Sulfonsäure.

Üblicherweise setzt man eine Verbindung der Formel (II), worin B⁻ für F⁻, Cl⁻, Br⁻, J⁻, HF₂⁻, BF₄⁻, C₆H₅SO₃⁻, p-CH₃-C₆H₅SO₃⁻, HSO₄⁻, PF₆⁻, CF₃SO₃⁻, insbesondere für F⁻, Cl⁻, Br⁻, J⁻, HF₂⁻, BF₄⁻, steht, ein.

Man setzt die Verbindung der Formel (II) in einer Menge von 0,5 bis 35, insbesondere 1 bis 30, bevorzugt 3 bis 25 Gew.-%, bezogen auf die Verbindung die gegen Fluor austauschbares Halogen enthält, ein.

Um nicht ausschließlich auf die vorstehenden Angaben in Gew.-% angewiesen zu sein, kann man in einer Vielzahl von Fällen, die Verbindung der Formel (II) in einer Menge von 0,1 bis 3, insbesondere von 0,4 bis 5, bevorzugt 0,5 bis 1 Mol-%, bezogen auf die Verbindung, die gegen Fluor austauschbares Halogen enthält, einsetzen. Diese Mengen erweisen sich üblicherweise als ausreichend.

Ohne Anspruch auf Vollständigkeit zu erheben, seien als Beispiele für Verbindungen der Formel (II) genannt.

Tetrakis(dimethylamino)phosphoniumchlorid Tetrakis(diethylamino)phosphoniumchlorid Tetrakis(dimethylamino)phosphoniumbromid Tetrakis(diethylamino)phosphoniumbromid Tetrakis(dipropylamino)phosphoniumchlorid oder -bromid Tris(diethylamino)(dimethylamino)phosphoniumchlorid oder -bromid Tetrakis(dibutylamino)phosphoniumchlorid oder -bromid Tris(dimethylamino)(diethylamino)phosphoniumchlorid oder -bromid Tris(dimethylamino)(cyclopentylamino)phosphoniumchlorid oder -bromid Tris(dimethylamino)(dipropylamino)phosphoniumchlorid oder -bromid Tris(dimethylamino)(dibutylamino)phosphoniumchlorid oder -bromid Tris(dimethylamino)(cyclohexylamino)phosphoniumchlorid oder -bromid Tris(dimethylamino)(diallylamino)phosphoniumchlorid oder -bromid Tris(dimethylamino)(dihexylamino)phosphoniumchlorid oder -bromid Tris(diethylamino)(dihexylamino)phosphoniumchlorid oder -bromid Tris(dimethylamino)(diheptylamino)phosphoniumchlorid oder -bromid Tris(diethylamino)(diheptylamino)phosphonium chlorid oder -bromid Tetrakis(pyrrollidino)phosphoniumchlorid oder -bromid Tetrakis(piperidino)phosphoniumchlorid oder -bromid Tetrakis(morpholino)phosphoniumchlorid oder -bromid Tris(piperidino)(diallylamino)phosphoniumchlorid oder -bromid Tris(pyrrolidino)(ethylmethylamino)phosphoniumchlorid oder -bromid Tris(pyrrollidino)(diethylamino)phosphoniumchlorid oder -bromid.

Man kann als Katalysator eine Verbindung der Formel (II) oder ein Gemisch zweier oder mehrerer Verbindungen der Formel (II) verwenden. Besonders einfach gestaltet sich dies, wenn man Gemische von Verbindungen der Formel (II), wie sie bei der Synthese anfallen, verwendet.

Das Verfahren läßt sich wie zuvor bereits erwähnt, in Anwesenheit oder Abwesenheit eines Lösungsmittels durchführen. Werden Lösungsmittel verwendet, so sind sowohl dipolar aprotische und aprotische als auch protische Lösungsmittel geeignet. Geeignete dipolar aprotische Lösungsmittel sind beispielsweise Dimethylsulfoxid (DMSO), Dimethylsulfon, Sulfolan (TMS), Dimethylformamid (DMFA), Dimethylacetamid, 1,3-Dimethylimidazolin-2-on, N-Methylpyrrolidon, Hexamethylphosphorsäuretriamid, Acetonitril und Benzonitril.
Diese Lösungsmittel können auch als Gemisch Anwendung finden.

Geeignete aprotische Lösungsmittel ohne ausgeprägten dipolaren Charakter sind aromatische Kohlenwasserstoffe oder chlorierte aromatische Kohlenwasserstoffe, beispielsweise Benzol, Toluol, ortho-, meta-, para-Xylol, technische Gemische isomerer Xylole, Ethylbenzol, Mesitylen, ortho-, meta-, para Chlortoluol, Chlorbenzol und ortho-, meta-, para-Dichlorbenzol. Es können auch Gemische dieser Lösungsmittel verwendet werden.

Das aprotische oder dipolar aprotische Lösungsmittel kann in beliebigen Mengen, beispielsweise 5 bis 500 Gew.-%, verwendet werden, bevorzugt werden allerdings geringe Mengen im Bereich von 5 bis 30 Gew.-%, bezogen auf die Verbindung, die gegen Fluor austauschbares Halogen enthält. Bei der Verwendung von protischen Lösungsmitteln liegen die eingesetzten Mengen im Bereich von 0,1 bis 5, bevorzugt 0,1 bis 2 Gew.-%, bezogen auf die Verbindung, die gegen Fluor austauschbares Halogen enthält.

Die Reaktionstemperatur hängt auch von der Art der Verbindung, die gegen Fluor austauschbares Halogen enthält, ab. So erfordern vergleichsweise reaktionsträge Verbindungen in der Regel höhere Reaktionstemperaturen, während vergleichsweise reaktive Ausgangsstoffe sich bereits bei relativ niedrigen Temperaturen mit Erfolg umsetzen lassen.

Gleiches trifft auch auf die Reaktionszeiten zu. Reaktionsträge Ausgangsstoffe erfordern in der Regel längere Reaktionszeiten als reaktivere Ausgangsstoffe.

An dieser Stelle sei darauf aufmerksam gemacht, daß ein Austausch lediglich eines Halogens gegen Fluor in der Regel einfacher durchzuführen ist, als ein Austausch von zwei oder mehreren Halogenen gegen Fluor. Der zweifache oder mehrfache Halogen-Fluor-Austausch erfordert, sofern er überhaupt abläuft, üblicherweise erheblich schärfere Reaktionsbedingungen (höhere Reaktionstemperaturen und längere Reaktionszeiten) als ein einfacher Halogen-Fluor-Austausch.

In einer Vielzahl von Fällen genügt es, das erfindungsgemäße Verfahren bei einer Temperatur von 60 bis 250, insbesondere 90 bis 220, bevorzugt 120 bis 200°C durchzuführen.

Das erfindungsgemäße Verfahren kann sowohl unter reduziertem Druck als auch unter Atmosphärendruck oder Überdruck ausgeübt werden. Diese Möglichkeit wird beispielsweise genutzt, indem geringe Mengen eines leicht siedenden aprotischen Lösungsmittels, das mit Wasser ein Azeotrop bildet, beispielsweise Benzol, Xylol, Mesitylen, Cyclohexan oder Toluol, vor Beginn der Reaktion in die Reaktionssuspension gegeben werden. Anschließend wird ein Teil des Lösungsmittels durch Anlegen eines reduzierten Druckes zusammen mit Wasser aus der Reaktionssuspension wieder entfernt. Durch diese Verfahrensweise läßt sich die Reaktionsgeschwindigkeit und die Ausbeute steigern und darüberhinaus die Bildung von Nebenprodukten minimieren.

Die Verbindung der Formel (II) kann in Abwesenheit oder Anwesenheit von Luftsauerstoff verwendet werden. Bevorzugt wird das Arbeiten unter Schutzgas beispielsweise Argon oder Stickstoff.

Bei Durchführung des Verfahrens ist zu gewährleisten, daß während der gesamten Reaktion das Reaktionsgemisch gut durchmischt wird.

Das Verfahren läßt sich diskontinuierlich oder kontinuierlich durchführen.

Die nachfolgenden Beispiele belegen die Erfindung ohne sie zu beschränken.

### Experimenteller Teil

### Herstellung von 4-Nitrofluorbenzol

### Beispiele 1 und 2

Herstellung von 4-Nitrofluorbenzol durch Umsetzung von 4-Nitrochlorbenzol mittels Tetrakis(dimethylamino)phosphoniumchlorid als Katalysator.

Man legt in einem 1,5 I Vierhalskolben, der mit Thermometer, Ankerrührer und Rückflußkühler mit Blasenzähler bestückt ist, 157 g (1 mol) 4-Nitrochlorbenzol, 400 ml Tetramethylsulfon (TMS), (Beispiel 1) respektive 340 ml Dimethylsulfoxid (DMSO) (Beispiel 2), 62,7 g (1,1 mol) Kaliumfluorid und 2,42 g (0,01 mol) Tetrakis(dimethylamino)phosphoniumchlorid vor. Anschließend erhitzt man unter Rühren auf die vorgegebene Reaktionstemperatur und läßt der vorgegebenen Zeit entsprechend reagieren.
Nach Beendigung der Umsetzung läßt man abkühlen, löst das Reaktionsgemisch in Methylenchlorid, filtriert unlösliche Bestandteile (Salze wie KCI, KF) ab und reinigt das Wertprodukt (4-Nitrofluorbenzol) durch fraktionierte Destillation unter reduziertem Druck.

### Vergleichsbeispiel 1

Herstellung von 4-Nitrofluorbenzol durch Umsetzung von 4-Nitrobenzol mittels Tetraphenylphosphoniumbromid als Katalysator

Man setzt 157 g (1 mol) 4-Nitrochlorbenzol, 400 ml Tetramethylensulfon, 62,7 g (1,1 mol) Kaliumfluorid jedoch 4,19 g (0,01 mol) Tetraphenylphosphoniumbromid ein und arbeitet wie in Beispiel 1 beschrieben.

### Beispiel 3

Herstellung von 4-Nitrofluorbenzol durch Umsetzung von 4-Nitrochlorbenzol mittels Tetrakis(diethylamino)phosphoniumbromid als Katalysator

Man legt in einem 500 ml Vierhalskolben, der mit Thermometer, Ankerrührer und Rückflußkühler mit Blasenzähler bestückt ist, 157 g (1 mol) 4-Nitrochlorbenzol, 74,1 g (1,3 mol) Kaliumfluorid und 3,99 g (0,01 mol) Tetrakis(diethylamino)-phosphoniumbromid, jedoch kein Lösungsmittel vor. Anschließend erhitzt man unter Rühren auf die vorgegebene Reaktionstemperatur und läßt der vorgegebenen Zeit entsprechend reagieren.
Die weitere Aufarbeitung erfolgt wie unter Beispiel 1 und 2 angegeben.

### Vergleichsbeispiel 2

Herstellung von 4-Nitrofluorbenzol durch Umsetzung von 4-Nitrochlorbenzol mittels Tetraphenylphosphoniumbromid-als Katalysator

Man arbeitet wie in Beispiel 3 angegeben, setzt jedoch anstatt desTetrakis(diethyl-amino)phosphoniumbromids 4,19 g (0,01 mol) Tetraphenylphosphoniumbromid ein.

Die Reaktionsbedingungen (Reaktionstemperatur, Zeit) sowie Umsatz und Ausbeute sind für die Beispiele 1 bis 3 und die Vergleichsbeispiele 1 und 2 der nachfolgenden Tabelle 1 zu entnehmen.

**Tabelle 1**

| Herstellung von 4-Nitrofluorbenzol | | | | | | | | |
|---|---|---|---|---|---|---|---|---|
| | 4-Nitrochlorbenzol | Lösungs mittel | KF | Katalysator | Zeit (Stunden) | Reaktions-temperatur | Umsatz % | Ausbeute % |
| Bsp. 1 | 1 mol | 400 ml | 1,1 | 0,01 | 5 | 180°C | 100 | 85 |
| | | TMS | mol | mol A | | | | |
| Vgl.-bsp. 1 | 1 mol | 400 ml | 1,1 | 0,01 | 5 | 180°C | 71 | 52 |
| | | TMS | mol | mol B | | | | |
| Bsp. 2 | 1 mol | 340 ml | 1,1 | 0,01 | 5 | 180°C | 100 | 86 |
| | | DMSO | mol | mol A | | | | |
| Bsp. 3 | 1 mol | - | 1,3 | 0,013 | 10 | 190°C | 99 | 76 |
| | | | mol | mol C | | | | |
| Vgl.-bsp. 2 | 1 mol | - | 1,3 | 0,013 | 10 | 190°C | 46 | 32 |
| | | | mol | mol B | | | | |
| TMS = Tetramethylensulfon (Sulfolan) | | | | | | | | |
| DMSO = Dimethylsulfoxid | | | | | | | | |
| Katalysator: A = Tetrakis(dimethylamino)phosphoniumchlorid [(CH₃)₂N]₄PCl B = Tetraphenylphosphoniumbromid (C₆H₅)₄PBr C = Tetrakis(diethylamino)phosphoniumbromid [(C₂H₅)₂N]₄PBr | | | | | | | | |

### Herstellung von 2-Nitrofluorbenzol

### Beispiele 4 und 5

### Herstellung von 2-Nitrofluorbenzol durch Umsetzung von 2-Nitrochlorbenzol mittels Tetrakis(diethylamino)phosphoniumbromid als Katalysator

Man legt in einem 1,5 I Vierhalskolben, der mit Thermometer, Ankerrührer und Rückflußkühler mit Blasenzähler bestückt ist, 157 g (1 mol) 2-Nitrochlorbenzol, 400 ml Dimethylsulfoxid (DMSO) (Beispiel 4) respektive 480 ml Tetramethylensulfon (TMS) (Beispiel 5), 68,4 g (1,2 mol) Kaliumfluorid und 3,99 g (0,01 mol) Tetrakis(diethylamino)phosphoniumbromid vor. Anschließend erhitzt man unter Rühren auf die vorgegebene Reaktionstemperatur und läßt der angegebenen Zeit entsprechend reagieren.
Die weitere Aufarbeitung erfolgt wie unter Beispiel 1 und 2 beschrieben.

### Vergleichsbeispiel 3

### Herstellung von 2-Nitrofluorbenzol durch Umsetzung von 2-Nitrochlorbenzol mittels Tetraphenylphosphoniumbromid als Katalysator

Man setzt 157 g (1 mol) 2-Nitrochlorbenzol, 400 ml Dimethylsulfoxid (DMSO), 68,4 g (1,2 mol) Kaliumfluorid und 4,19 g (0,01 mol) Tetraphenylphosphoniumbromid ein und arbeitet wie in Beispiel 4 beschrieben.

### Vergleichsbeispiel 4

### Herstellung von 2-Nitrofluorbenzol durch Umsetzung von 2-Nitrochlorbenzol mittels 18-Krone-6-ether als Katalysator

Man setzt 157 g (1 mol) 2-Nitrochlorbenzol, 480 ml Dimethylsulfoxid, 68,4 g (1,2 mol) Kaliumfluorid und 2,64 g (0,01 mol) 18-Krone-6-ether ein und arbeitet wie in Beispiel 5 beschrieben.

### Beispiel 6

### Herstellung von 2-Nitrofluorbenzol durch Umsetzung von 2-Nitrochlorbenzol mittels Tetrakis(diethylamino)phosphoniumbromid als Katalysator.

Man legt in einem 500 ml Vierhalskolben, der mit Thermometer, Ankerrührer und Rückflußkühler mit Blasenzähler bestückt ist, 157 g (1 mol) 2-Nitrochlorbenzol, 68,4 g (1,2 mol) Kaliumfluorid und 3,99 g (0,01 mol) Tetrakis(diethylamino)-phosphoniumbromid, jedoch kein Lösungsmittel, vor. Anschließend erhitzt man unter Rühren auf die vorgegebene Reaktionstemperatur und läßt der angegebenen Zeit entsprechend reagieren.
Die weitere Aufarbeitung erfolgt wie unter Beispiel 1 und 2 beschrieben.

### Vergleichsbeispiel 5

### Herstellung von 2-Nitrofluorbenzol durch Umsetzung von 2-Nitrochlorbenzol mittels Tetraphenylphosphoniumbromid als Katalysator

Man arbeitet wie in Beispiel 6 angegeben, setzt jedoch anstatt Tetrakis(diethylamino)phosphoniumbromid 4,19 g (0,01 mol) Tetraphenylphosphoniumbromid ein.

Die Reaktionsbedingungen (Reaktionstemperatur, Zeit) sowie Umsatz und Ausbeute sind für die Beispiele 4 bis 6 und die Vergleichsbeispiele 4 und 5 der nachfolgenden Tabelle 2 zu entnehmen.

**Tabelle 2**

| Herstellung von 2-Nitrofluorbenzol | | | | | | | | |
|---|---|---|---|---|---|---|---|---|
| | 2-Nitrochlorbenzol | Lösungs mittel | KF | Katalysator | Zeit (Stunden) | Reaktions-temperatur | Umsatz % | Ausbeute % |
| Bsp. 4 | 1 mol | 400 ml | 1,2 | 0,01 | 5 | 180°C | 98 | 74 |
| | | DMSO | mol | mol C | | | | |
| Vgl.-bsp. 3 | 1 mol | 400 ml | 1,2 | 0,01 | 5 | 180°C | 42 | 31 |
| | | DMSO | mol | mol B | | | | |
| Bsp. 5 | 1 mol | 480 ml | 1,2 | 0,01 | 7 | 180°C | 100 | 85 |
| | | TMS | mol | mol C | | | | |
| Vgl.-bsp. 4 | 1 mol | 480 ml | 1,2 | 0,01 | 7 | 180°C | 33 | 20 |
| | | TMS | mol | mol D | | | | |
| Bsp. 6 | 1 mol | - | 1,2 | 0,01 | 10 | 190°C | 99 | 71 |
| | | | mol | mol C | | | | |
| Vgl.-bsp. 5 | 1 mol | - | 1,2 | 0,01 | 10 | 190°C | 42 | 30 |
| | | | mol | mol B | | | | |
| DMSO = Dimethylsulfoxid | | | | | | | | |
| TMS = Tetramethylensulfon (Sulfolan) | | | | | | | | |
| Katalysator: C = Tetrakis(diethylamino)phosphoniumbromid [(C₂H₅)₂N]₄PBr B = Tetraphenylphosphoniumbromid (C₆H₅)₄PBr D = 18-Krone-6-ether | | | | | | | | |

### Herstellung von 2,4-Difluomitrobenzol

### Beispiele 7 und 8

### Herstellung von 2,4-Difluornitrobenzol durch Umsetzung von 2,4-Dichlomitrobenzol

Man legt in einem 1,5 I Vierhalskolben, der mit Thermometer, Ankerrührer und Rückflußkühler mit Blasenzähler bestückt ist, 192 g (1 mol) 2,4-Dichlomitrobenzol, 550 ml Tetramethylensulfon (TMS), 136,8 g (2,4 mol) Kaliumfluorid und 5,99 g (0,015 mol) Tetrakis(diethylamino)phosphoniumbromid (Beispiel 7) respektive 3,63g (0,015 mol) Tetrakis(dimethylamino)phosphoniumchlorid (Beispiel 8) vor. Anschließend erhitzt man unter Rühren auf die vorgegebene Reaktionstemperatur und läßt der angegebenen Zeit entsprechend reagieren.
Die weitere Aufarbeitung erfolgt wie unter Beispiel 1 und 2 beschrieben.

### Vergleichsbeispiel 6

### Herstellung von 2,4-Difluornitrobenzol durch Umsetzung von 2,4-Dichlornitrobenzol mittels Tetraphenylphosphoniumbromid als Katalysator

Man setzt 192 g (1 mol) 2,4-Dichlornitrobenzol, 550 ml Tetramethylensulfon, 136,8 g (2,4 mol) Kaliumfluorid und 6,29 g (0,015 mol) Tetraphenylphosphoniumbromid ein und arbeitet wie in Beispiel 7 und 8 beschrieben.

### Beispiele 9 und 10

### Herstellung von 2,4-Difluomitrobenzol durch Umsetzung von 2,4-Dichlornitrobenzol

Man legt in einem 500 ml Vierhalskolben, der mit Thermometer, Ankerrührer und Rückflußkühler mit Blasenzähler bestückt ist, 192 g (1 mol) 2,4-Dichlornitrobenzol, 136,8 g (2,4 mol) Kaliumfluorid, 7,98 g (0,02 mol) Tetrakis(diethylamino)-phosphoniumbromid (Beispiel 9) respektive 8,54g (0,02 mol) Ethyl-butyl-aminotris(diethylamino)phosphoniumbromid (Beispiel 10), jedoch kein Lösungsmittel vor. Anschließend erhitzt man unter Rühren auf die vorgegebene Reaktionstemperatur und läßt der angegebenen Zeit entsprechend reagieren.
Die weitere Aufarbeitung erfolgt wie unter Beispiel 1 und 2 beschrieben.

### Vergleichsbeispiel 7

### Herstellung von 2,4-Difluornitrobenzol durch Umsetzung von 2,4-Dichlornitrobenzol mittels Tetraphenylphosphoniumbromid als Katalysator (ohne Lösungsmittel)

Man arbeitet wie in Beispiel 8 und 9 angegeben, setzt jedoch anstatt Tetrakis(diethylamino)phosphoniumbromid respektive Ethyl-butyl-amino-tris-(diethylamino)phosphoniumbromid 8,38 g (0,02 mol) Tetraphenylphosphoniumbromid ein.

Die Reaktionsbedingungen (Reaktionstemperatur, Zeit) sowie Umsatz und Ausbeute sind für die Beispiele 7 bis 10 und die Vergleichsbeispiele 6 und 7 der nachfolgenden Tabelle 3 zu entnehmen.

**Tabelle 3**

| Herstellung von 2,4-Difluornitrobenzol | | | | | | | | |
|---|---|---|---|---|---|---|---|---|
| | 2,4-Dichlornitrobenzol | Lösungs mittel | KF | Katalysator | Zeit (Stunden) | Reaktions-temperatur | Umsatz % | Ausbeute % |
| Bsp. 7 | 1 mol | 500 ml | 2,4 | 0,015 | 6 | 180°C | 99 | 75 |
| | | TMS | mol | mol C | | | | |
| Bsp. 8 | 1 mol | 550 ml | 2,4 | 0,015 | 6 | 180°C | 97 | 78 |
| | | TMS | mol | mol A | | | | |
| Vgl.-bsp. 6 | 1 mol | 550 ml | 2,4 | 0,015 | 6 | 180°C | 73 | 51 |
| | | TMS | mol | mol B | | | | |
| Bsp. 9 | 1 mol | - | 2,4 | 0,02 | 10 | 190°C | 100 | 69 |
| | | | mol | mol C | | | | |
| Bsp.10 | 1 mol | - | 2,4 | 0,02 | 10 | 190°C | 100 | 75 |
| | | | mol | mol E | | | | |
| Vgl.-bsp. 7 | 1 mol | - | 2,4 | 0,02 | 10 | 190°C | 48 | 35 |
| | | | mol | mol B | | | | |
| TMS = Tetramethylensulfon (Sulfolan) | | | | | | | | |
| Katalysator: A = Tetrakis(dimethylamino)phosphoniumchlorid ((CH₃)₂N]₄PCl B = Tetraphenylphosphoniumbromid (C₆H₅)₄PBr C = Tetrakis(diethylamino)phosphoniumbromid [(C₂H₅)₂N]₄PBr E = Ethyl-butyl-amino-tris(diethylamino)phosphoniumbromid | | | | | | | | |

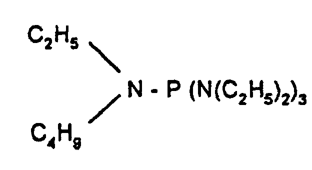

Herstellung von 2,3-Difluor-5-chlorpyridin

### Beispiel 11

### Herstellung von 2,3-Difluor-5-chlorpyridin durch Umsetzung von 2-Fluor-3,5-dichlorpyridin mittels Tetrakis(diethylamino)phosphoniumbromid

Man legt in einem 500 ml Vierhalskolben, der mit Thermometer, Ankerrührer und Rückflußkühler mit Blasenzähler bestückt ist, 166 g (1 mol) 2-Fluor-3,5-dichlorpyridin, 68,4 g (1,2 mol) Kaliumfluorid und 3,99 g (0,01 mol) Tetrakis(diethylamino)phosphoniumbromid vor. Anschließend erhitzt man unter Rühren auf die vorgegebene Reaktionstemperatur und läßt der angegebenen Zeit entsprechend reagieren. Nach Beendigung der Umsetzung läßt man abkühlen, gießt das Reaktionsgemisch in Wasser, das im Überschuß eingesetzt wird, extrahiert mit Methylenchlorid, wäscht die abgetrennte Methylenchloridphase mit Wasser, trocknet sie und destilliert anschließend fraktioniert unter reduziertem Druck.

### Vergleichsbeispiel 8

### Herstellung von 2,3-Difluor-5-chlorpyridin durch Umsetzung von 2-Fluor-3,5-dichlorpyridin mittels Tetraphenylphosphoniumbromid als Katalysator

Man setzt 166 g (1 mol) 2-Fluor-3,5-dichlorpyridin, 68,4 g (1,2 mol) Kaliumfluorid und 4,19 g (0,01 mol) Tetraphenylphosphoniumbromid ein und arbeitet wie in Beispiel 11 beschrieben.
Die Reaktionsbedingungen (Reaktionstemperatur, Zeit) sowie Umsatz und Ausbeute sind für Beispiel 11 und Vergleichsbeispiel 8 der nachfolgenden Tabelle 4 zu entnehmen.

Herstellung von 1-Fluor-3,5-dichlorbenzol und 1,3-Difluor-5-chlorbenzol

### Beispiel 12

### Herstellung von 1-Fluor-3,5-dichlorbenzol und 1,3-Difluor-5-chlorbenzol durch Umsetzung von 1,3,5-Trichlorbenzol mittels Tetrakis(diethylamino)-phosphoniumbromid

Man legt in einem 500 ml Vierhalskolben, der mit Thermometer, Ankerrührer und Rückflußkühler mit Blasenzähler bestückt ist, 181,5 g (1 mol) 1,3,5-Trichlorbenzol, 136,8 g (2,4 mol) Kaliumfluorid und 7,98 g (0,02 mol) Tetrakis(diethylamino)-phosphoniumbromid vor. Anschließend erhitzt man unter Rühren auf die vorgegebene Reaktionstemperatur und läßt der angegebenen Zeit entsprechend reagieren. Nach Beendigung der Umsetzung läßt man abkühlen, löst das Reaktionsgemisch in Methylenchlorid, filtriert unlösliche Bestandteile (Salze wie KCl, KF) ab und reinigt die Wertprodukte (1-Fluor-3,5-dichlorbenzol und 1,3-Difluor-5-chlorbenzol) durch fraktionierte Destillation.

Die Reaktionsbedingungen (Reaktionstemperatur, Zeit) sowie Umsatz und Ausbeute sind für Beispiel 12 der nachfolgenden Tabelle 4 zu entnehmen.

Herstellung von 1,2,3,4-Tetrafluorbenzotrifluorid

### Beispiel 13

### Herstellung von 1,2,3,4-Tetrafluorbenzotrifluorid durch Umsetzung von 1,2,3,4-Tetrachlorbenzotrifluorid mittels Tetrakis(diethylamino)phosphoniumbromid als Katalysator

Man legt in einem 1000 ml Vierhalskolben, der mit Thermometer, Ankerrührer und Rückflußkühler mit Blasenzähler bestückt ist, 284 g (1 mol) 1,2,3,4-Tetrachlorbenzotrifluorid, 285 g (5 mol) Kaliumfluorid und 15,96 g (0,04 mol) Tetrakis(diethylamino)-phosphoniumbromid vor. Anschließend erhitzt man unter Rühren auf die vorgegebene Reaktionstemperatur und läßt der angegebenen Zeit entsprechend reagieren.
Nach Beendigung der Umsetzung läßt man abkühlen, löst das Reaktionsgemisch in Methylenchlorid, filtriert von unlöslichen Bestandteilen (Salze wie KCI, KF) ab und reinigt das Wertprodukt (1,2,3,4-Tetrafluorbenzotrifluorid) durch fraktionierte Destillation.

### Vergleichsbeispiel 9

### Herstellung von 1,2,3,4-Tetrafluorbenzotrifluorid durch Umsetzung von 1,2,3,4-Tetrachlorbenzotrifluorid mittels Tetraphenylphosphoniumbromid als Katalysator.

Man setzt 284 g (1 mol) 1,2,3,4-Tetrachlorbenzotrifluorid, 285g (5 mol) Kaliumfluorid und 16,76 g (0,04 mol) Tetraphenylphosphoniumbromid ein und arbeitet wie in Beispiel 13 beschrieben.

Die Reaktionsbedingungen (Reaktionstemperatur, Zeit) sowie Umsatz und Ausbeute sind für Beispiel 13 und Vergleichsbeispiel 9 der nachfolgenden Tabelle 4 zu entnehmen.

**Tabelle 4**

| Herstellung von 2,3-Difluor-5-chlorpyridin, 1-Fluor-3,5-dichlorbenzol, 1,3-Difluor-5-chlorbenzol und 1,2,3,4-Tetrafluorbenzotrifluorid ohne Zusatz eines Lösungsmittels | | | | | | | |
|---|---|---|---|---|---|---|---|
| | Einsatzstoff | KF | Katalysator | Zeit (Stunden) | Reaktions-temperatur | Umsatz % | Ausbeute % |
| Bsp.11 | 1 mol | 1,2 | 0,01 | 4 | 170°C | 55 | 40 |
| | 2-Fluor-3,5- | mol | mol C | | | | |
| | dichlorpyridin | | | | | | |
| Vgl.-bsp. 8 | 1 mol | 1,2 | 0,01 | 4 | 170°C | 5 | 1-2 |
| | 2-Fluor-3,5- | mol | mol B | | | | |
| | dichlorpyridin | | | | | | |
| Bsp.12 | 1 mol | 2,4 | 0,02 | 10 | 180°C | 100 | 50* |
| | 1,3,5-Tri- | mol | mol C | | | | 37** |
| | chlorbenzol | | | | | | |
| Bsp.13 | 1 mol | 5 mol | 0,04 | 10 | 190°C | 96 | 68 |
| | 1,2,3,4-Tetra- | | mol C | | | | |
| | chlorbenzo- | | | | | | |
| | trifluorid | | | | | | |
| Vgl.-bsp. 9 | 1 mol | 5 mol | 0,04 | 10 | 190°C | 35 | 20 |
| | 1,2,3,4-Tetra- | | mol B | | | | |
| | chlorbenzo- | | | | | | |
| | trifluorid | | | | | | |

| | | | | | | | |
|---|---|---|---|---|---|---|---|
| * Ausbeute an 1-Fluor-3,5-dichlorbenzol | | | | | | | |
| ** Ausbeute an 1,3-Difluor-5-chlorbenzol | | | | | | | |
| Katalysator: B = Tetraphenylphosphoniumbromid (C₆H₅)₄PBr | | | | | | | |
| C = Tetrakis(diethylamino)phosphoniumbromid [(C₂H₅)₂N]₄PBr | | | | | | | |

## Patentansprüche

1. Verfahren zur Herstellung von Fluor enthaltenden Verbindungen, durch Umsetzung einer Verbindung, die gegen Fluor austauschbares Halogen enthält, mit einem Fluorid oder einem Gemisch von Fluoriden der allgemeinen Formel I
MeF (I),
worin Me für ein Erdalkalimetallion, NH₄⁺-ion oder Alkalimetallion steht, in Anwesenheit oder Abwesenheit eines Lösungsmittels bei einer Temperatur von 40 bis 260°C, **dadurch gekennzeichnet, daß** man die Umsetzung in Anwesenheit einer Verbindung oder eines Gemisches von Verbindungen der allgemeinen Formel (II) 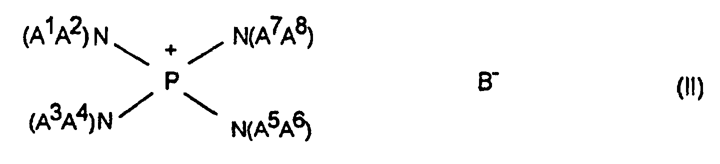
worin A¹, A², A³, A⁴, A⁵, A⁶, A⁷, A⁸ unabhängig voneinander, gleich oder verschieden sind und für ein geradkettiges oder verzweigtes Alkyl oder Alkenyl mit 1 bis 12 Kohlenstoffatomen, Cycloalkyl mit 4 bis 8 Kohlenstoffatomen, für ein Aryl mit 6 bis 12 Kohlenstoffatomen, ein Aralkyl mit 7 bis 12 Kohlenstoffatomen stehen, oder A¹ A², A³A⁴, A⁵A⁶, A⁷A⁸ unabhängig voneinander, gleich oder verschieden sind und direkt oder über O oder N-A⁹ miteinander zu einem Ring mit 3 bis 7 Ringgliedern verbunden sind, A⁹ für ein Alkyl mit 1 bis 4 Kohlenstoffatomen steht und B⁻ einen einwertigen Säurerest oder das Äquivalent eines mehrwertigen Säurerests bedeutet, durchführt.

2. Verfahren nach Anspruch 1, **dadurch gekennzeichnet, daß** man als Verbindung, die gegen Fluor austauschbares Halogen enthält, eine am Kern einen gegen Fluor austauschbaren Chlor- oder Bromsubstituenten besitzende aromatische Verbindung mit 0 bis 3 Stickstoffatome im Kern, die gegebenenfalls wenigstens einen weiteren, die nucleophile Substitution der aromatischen Verbindungen begünstigenden Substituenten aufweist, einsetzt.

3. Verfahren nach Anspruch 1 oder 2, **dadurch gekennzeichnet, daß** man eine am Kern einen gegen Fluor austauschbaren Chlor- oder Bromsubstituenten besitzende aromatische Verbindung, die wenigstens einen weiteren Substituenten aus der Reihe F, Cl, Br, J, NO₂, CF₃, CN, CHO, COF, COCI, SO₂F, SO₂Cl, OCF₃, SCF₃, SOCF₃, SO₂CF₃, COOR, CONRR', SO₂R, COR oder OR oder einen Rest -CO-O-CO-, -CO-NR-CO-, der zwei ortho-Stellungen verknüpft, aufweist, wobei R und R' jeweils unabhängig voneinander, gleich oder verschieden sind, und für H, ein geradkettiges oder verzweigtes Alkyl mit 1 bis 6 Kohlenstoffatomen, ein Aryl mit 6 bis 12 Kohlenstoffatomen oder Aralkyl mit 7 bis 12 Kohlenstoffatomen stehen und die Alkyle und Aralkyle gegebenenfalls einfach bis dreifach halogensubstituiert sind, einsetzt.

4. Verfahren nach einem oder mehreren der Ansprüche 1 bis 3, **dadurch gekennzeichnet, daß** man eine am Kern einen gegen Fluor austauschbaren Chloroder Bromsubstituenten besitzende aromatische Verbindung, die wenigstens ein gegen Fluor austauschbares Chlor oder Brom als weiteren Substituenten und gegebenenfalls wenigstens einen weiteren Substituenten aus der Reihe F, NO₂, CF₃, CN, CHO, COF, COCI, SO₂F, SO₂Cl, OCF₃, SCF₃, SO₂CF₃, COOR, CONRR', SO₂R, COR, OR, -CO-O-CO- oder -CO-NR-CO- besitzt, einsetzt.

5. Verfahren nach einem oder mehreren der Ansprüche 1 bis 4, **dadurch gekennzeichnet, daß** man eine Verbindung der allgemeinen Formel (III) 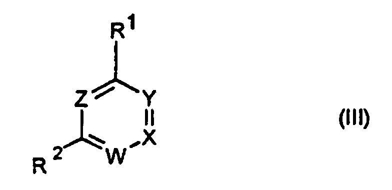 worin W für N oder C-R³, X für N oder C-R⁴, Y für N oder C-R⁵, Z für N oder C-R⁶ steht, W, X und Y nicht zugleich N sind, R¹, R², R³, R⁴, R⁵, R⁶ gleich oder verschieden sind und H, F, Cl, Br, J, NO₂, CF₃, CN, CHO, COF, COCl, SO₂F, SO₂Cl, OCF₃, SCF₃, SO₂CF₃, COOR, CONRR', SO₂R, COR, OR, einen Rest -CO-O-CO-, -CO-NR-CO- oder -CR"=CR"-CR"=CR"-, der zwei ortho-Stellungen verknüpft, bedeuten, R und R' die vorstehende Bedeutung besitzen und R" unabhängig voneinander, gleich oder verschieden sind und dieselbe Bedeutung wie R¹ bis R⁶ haben, und wenigstens einer der Reste R¹ bis R⁶ Chlor oder Brom, einsetzt.

6. Verfahren nach einem oder mehreren der Ansprüche 1 bis 5, **dadurch gekennzeichnet, daß** man eine Verbindung der Formel (III), worin nur einer der Reste R¹ bis R⁶ Chlor oder Brom ist, keiner der Reste W, X, Y, Z für ein Stickstoffatom steht und wenigstens einer der verbleibenden Reste aus der Gruppe R¹ bis R⁶ NO₂, CF₃, CN, CHO, COF, COCl, SO₂F, SO₂Cl, OCF₃, SCF₃, SO₂CF₃, COOR, COONRR', SO₂R, COR, OR, -CO-O-CO-, -CO-NR-CO- oder -CR"=CR"-CR"=CR"- ist, einsetzt.

7. Verfahren nach einem oder mehreren der Ansprüche 1 bis 5, **dadurch gekennzeichnet, daß** man eine Verbindung der Formel (III), worin zwei oder mehrere der Reste R¹ bis R⁶ Chlor oder Brom sind, die Reste W, X, Y, Z für 0 bis 3 Stickstoffatome stehen und die verbleibenden Reste aus der Gruppe R¹ bis R⁶ alle Wasserstoff sein können, einsetzt.

8. Verfahren nach einem oder mehreren der Ansprüche 1 bis 7, **dadurch gekennzeichnet, daß** man als Fluorid der Formel (I) Lithiumfluorid, Natriumfluorid, Kaliumfluorid, Rubidiumfluorid, Cäsiumfluorid oder ein Gemisch derselben einsetzt.

9. Verfahren nach einem oder mehreren der Ansprüche 1 bis 8, **dadurch gekennzeichnet, daß** man das Fluorid der Formel (II): Äquivalent auszutauschendes Halogen im Verhältnis (0,5 bis 10):(1), insbesondere (0,8 bis 5): (1), bevorzugt (1 bis 2):(1) einsetzt.

10. Verfahren nach einem oder mehreren der Ansprüche 1 bis 9, **dadurch gekennzeichnet, daß** man eine Verbindung der Formel (II), worin A¹, A², A³, A⁴, A⁵, A⁶, A⁷, A⁸ unabhängig voneinander, gleich oder verschieden sind und für ein geradkettiges oder verzweigtes Alkyl oder Alkenyl mit 1 bis 12 Kohlenstoffatomen oder Cycloalkyl mit 4 bis 8 Kohlenstoffatomen, stehen, einsetzt.

11. Verfahren nach einem oder mehreren der Ansprüche 1 bis 10, **dadurch gekennzeichnet, daß** man eine Verbindung der Formel (II), worin A¹, A², A³, A⁴, A⁵, A⁶, A⁷, A⁸ unabhängig voneinander, gleich oder verschieden sind und für ein geradkettiges oder verzweigtes Alkyl oder Alkenyl mit 1 bis 8 Kohlenstoffatomen oder Cycloalkyl mit 5 bis 6 Kohlenstoffatomen, stehen, einsetzt.

12. Verfahren nach einem oder mehreren der Ansprüche 1 bis 11, **dadurch gekennzeichnet, daß** man eine Verbindung der Formel (II), worin A¹, A², A³, A⁴, A⁵, A⁶, A⁷, A⁸ unabhängig voneinander, gleich oder verschieden sind und für ein geradkettiges oder verzweigtes Alkyl oder Alkenyl mit 1 bis 4 Kohlenstoffatomen stehen, einsetzt.

13. Verfahren nach einem oder mehreren der Ansprüche 1 bis 12, **dadurch gekennzeichnet, daß** man eine Verbindung der Formel (II), worin A¹A² = A³A⁴ oder A¹A² = A³A⁴ = A⁵A⁶ oder A¹A² =A³A⁴ = A⁵A⁶ = A⁷A⁸ ist, einsetzt.

14. Verfahren nach einem oder mehreren der Ansprüche 1 bis 13, **dadurch gekennzeichnet, daß** man eine Verbindung der Formel (II), worin A¹ = A² = A³ = A⁴ oder A¹ = A² = A³ = A⁴ = A⁵ = A⁶ oder A¹ = A² = A³ = A⁴ = A⁵ = A⁶ = A⁷ = A⁸ ist, einsetzt.

15. Verfahren nach einem oder mehreren der Ansprüche 1 bis 14, **dadurch gekennzeichnet, daß** man eine Verbindung der Formel (II), worin A¹A² oder A¹A² und A³A⁴ oder A¹A² und A³A⁴ und A⁵A⁶ oder A¹A² und A³A⁴ und A⁵A⁶ und A⁷A⁸ direkt oder über O oder N-A⁹ miteinander zu einem gesättigten oder ungesättigten Ring mit 5 oder 6 Ringgliedern verbunden sind, einsetzt.

16. Verfahren nach einem oder mehreren der Ansprüche 1 bis 6 und 15, **dadurch gekennzeichnet, daß** man eine Verbindung der Formel (II), worin A¹A² oder A¹A² und A³A⁴ oder A¹A² und A³A⁴ und A5A6 oder A¹A² und A³A⁴ und A⁵A⁶ und A⁷A⁸ zu einem Ring, der das N-Atom, an dem die jeweiligen Reste A¹ bis A⁸ sitzen, gegebenenfalls O oder N-A⁹ und CH₂-Gruppen als Ringglieder umfaßt, verbunden sind, einsetzt.

17. Verfahren nach einem oder mehreren der Ansprüche 1 bis 16, **dadurch gekennzeichnet, daß** man eine Verbindung der Formel (II), worin B⁻ für F⁻, Cl⁻, Br⁻, HF₂⁻, J⁻, BF₄⁻, C₆H₅SO₃⁻, p-CH₃-C₆H₄-SO₃⁻, HSO₄⁻, PF₆⁻, CF₃SO₃⁻ steht, einsetzt.

18. Verfahren nach einem oder mehreren der Ansprüche 1 bis 17, **dadurch gekennzeichnet, daß** man eine Verbindung der Formel (II), worin B⁻ für F⁻, Cl⁻, Br⁻, HF₂⁻, BF₄⁻ steht, einsetzt.

19. Verfahren nach einem oder mehreren der Ansprüche 1 bis 18, **dadurch gekennzeichnet, daß** man die Verbindung der Formel (II) in einer Menge von 0,5 bis 35 Gew.-%, bezogen auf die Verbindung, die gegen Fluor austauschbares Halogen enthält, einsetzt.

20. Verfahren nach einem oder mehreren der Ansprüche 1 bis 19, **dadurch gekennzeichnet, daß** man als Lösungsmittel ein dipolar aprotisches, ein aprotisches oder ein protisches Lösungsmittel einsetzt.

21. Verfahren nach einem oder mehreren der Ansprüche 1 bis 20, **dadurch gekennzeichnet, daß** man als dipolar aprotisches Lösungsmittel Dimethylsulfoxid, Dimethylsulfon, Sulfolan, Dimethylformamid, Dimethylacetamid, 1,3-Dimethylimidazolin-2-on, N-Methylpyrrolidon, Hexamethylphosphorsäuretriamid, Acetonitril, Benzonitril oder ein Gemisch dieser Lösungsmittel einsetzt.

22. Verfahren nach einem oder mehreren der Ansprüche 1 bis 20, **dadurch gekennzeichnet, daß** man als aprotisches Lösungsmittel einen aromatischen Kohlenwasserstoff, einen chlorierten aromatischen Kohlenwasserstoff oder ein Gemisch dieser Lösungsmittel einsetzt.

23. Verfahren nach einem oder mehreren der Ansprüche 1 bis 20 und 22, **dadurch gekennzeichnet, daß** man als aprotisches Lösungsmittel Benzol, Toluol, ortho-, meta-, para-Xylol, technische Gemische isomerer Xylole, Ethylbenzol, Mesitylen, ortho-, meta-, para-Chlortoluol, Chlorbenzol, ortho-, meta-, para-Dichlorbenzol oder ein Gemisch dieser Lösungsmittel einsetzt.

24. Verfahren nach einem oder mehreren der Ansprüche 1 bis 20, **dadurch gekennzeichnet, daß** man als protisches Lösungsmittel Methanol, Ethanol, Propanol, Isopropanol, Butanol, Isobutanol, Polyalkylenglykole mit Ethylen-, Propylen- oder Butylen-Einheiten oder ein Gemisch dieser Lösungsmittel einsetzt.

25. Verfahren nach einem oder mehreren der Ansprüche 1 bis 24, **dadurch gekennzeichnet, daß** man die Umsetzung bei 60 bis 250°C durchführt.

26. Verfahren nach einem oder mehreren der Ansprüche 1 bis 25, **dadurch gekennzeichnet, daß** man die Umsetzung bei 90 bis 220°C durchführt.

27. Verfahren nach einem oder mehreren der Ansprüche 1 bis 26, **dadurch gekennzeichnet, daß** man die Umsetzung bei 120 bis 200°C durchführt.

## Claims

1. A process for preparing fluorine-containing compounds by reacting a compound which contains fluorine-exchangeable halogen with a fluoride or a mixture of fluorides with the formula I
MeF (I),
in which Me is an alkaline earth metal ion, NH₄⁺ ion or alkali metal ion, in the presence or absence of a solvent at a temperature of from 40 to 260°C, which comprises carrying out the reaction in the presence of a compound or a mixture of compounds of the formula (II) 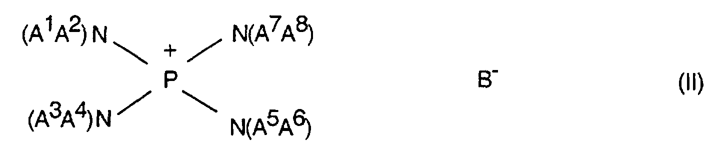
in which A¹, A², A³, A⁴, A⁵, A⁶, A⁷ and A⁸ independently of one another are identical or different and are a straight-chain or branched alkyl or alkenyl having 1 to 12 carbon atoms, cycloalkyl having 4 to 8 carbon atoms, an aryl having 6 to 12 carbon atoms, or an aralkyl having 7 to 12 carbon atoms, or A¹ A², A³A⁴, A⁵A⁶ and A⁷A⁸ independently of one another are identical or different and are connected to one another directly or by way of O or N-A⁹ to form a ring having 3 to 7 ring members, A⁹ is an alkyl having 1 to 4 carbon atoms and B⁻ is a monovalent acid radical or the equivalent of a polyvalent acid radical.

2. The process as claimed in claim 1, wherein the compound employed which contains fluorine-exchangeable halogen is an aromatic compound whose ring system has from 0 to 3 nitrogen atoms and carries a chlorine or bromine substituent which can be exchanged for fluorine, which compound may have at least one further substituent which promotes the nucleophilic substitution of the aromatic compounds.

3. The process as claimed in claim 1 or 2, wherein an aromatic compound is employed which has on the ring system a fluorine-exchangeable chlorine or bromine substituent and has at least one further substituent from the series consisting of F, Cl, Br, I, NO₂, CF₃, CN, CHO, COF, COCI, SO₂F, SO₂Cl, OCF₃, SCF₃, SOCF₃, SO₂CF₃, COOR, CONRR', SO₂R, COR, OR or a radical -CO-O-CO-, -CO-NR-CO-, which links two ortho positions, where R and R' each independently of one another are identical or different and are H, a straight-chain or branched alkyl having 1 to 6 carbon atoms, an aryl having 6 to 12 carbon atoms or aralkyl having 7 to 12 carbon atoms and where the alkyls and aralkyls are unsubstituted or substituted from one to three times by halogen.

4. The process as claimed in one or more of claims 1 to 3, wherein an aromatic compound is employed which has on the ring system a fluorine-exchangeable chlorine or bromine substituent and has at least one fluorine-exchangeable chlorine or bromine as further substituent and may have at least one further substituent from the series consisting of F, NO₂, CF₃, CN, CHO, COF, COCI, SO₂F, SO₂Cl, OCF₃, SCF₃, SO₂CF₃, COOR, CONRR', SO₂R, COR, OR, -CO-O-CO- and -CO-NR-CO-.

5. The process as claimed in one or more of claims 1 to 4, wherein a compound of the formula (III) is employed 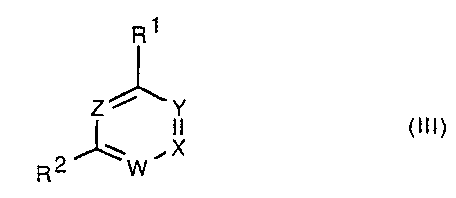 in which W is N or C-R³, X is N or C-R⁴, Y is N or C-R⁵, Z is N or C-R⁶ but, W, X and Y are not simultaneously N, R¹, R², R³, R⁴, R⁵ and R⁶ are identical or different and are H, F, Cl, Br, I, NO₂, CF₃, CN, CHO, COF, COCl, SO₂F, SO₂Cl, OCF₃, SCF₃, SO₂CF₃, COOR, CONRR', SO₂R, COR, OR or a radical -CO-O-CO-, -CO-NR-CO- or -CR"=CR"-CR"=CR"-, which links two ortho positions, R and R' are as defined above and radicals R" independently of one another are identical or different and have the same meaning as R¹ to R⁶, and at least one of the radicals R¹ to R⁶ is chlorine or bromine.

6. The process as claimed in one or more of claims 1 to 5, wherein a compound of the formula (III) is employed in which only one of the radicals R¹ to R⁶ is chlorine or bromine, none of the radicals W, X, Y and Z is a nitrogen atom and at least one of the remaining radicals from the group R¹ to R⁶ is NO₂, CF₃, CN, CHO, COF, COCI, SO₂F, SO₂Cl, OCF₃, SCF₃, SO₂CF₃, COOR, COONRR', SO₂R, COR, OR, -CO-O-CO-, -CO-NR-CO- or -CR"=CR"-CR"=CR"-.

7. The process as claimed in one or more of claims 1 to 5, wherein a compound of the formula (III) is employed in which two or more of the radicals R¹ to R⁶ are chlorine or bromine, radicals W, X, Y and Z are from 0 to 3 nitrogen atoms and the remaining radicals from the group R¹ to R⁶ can all be hydrogen.

8. The process as claimed in one or more of claims 1 to 7, wherein the fluoride of the formula (I) employed is lithium, sodium, potassium, rubidium or cesium fluoride or a mixture thereof.

9. The process as claimed in one or more of claims 1 to 8, wherein the ratio of the fluoride of the formula (II) to equivalent of halogen to be exchanged is (0.5 to 10): (1), especially (0.8 to 5): (1), preferably (1 to 2):(1).

10. The process as claimed in one or more of claims 1 to 9, wherein a compound of the formula (II) is employed in which A¹, A², A³, A⁴, A⁵, A⁶, A⁷ and A⁸ independently of one another are identical or different and are a straight-chain or branched alkyl or alkenyl having 1 to 12 carbon atoms or cycloalkyl having 4 to 8 carbon atoms.

11. The process as claimed in one or more of claims 1 to 10, wherein a compound of the formula (II) is employed in which A¹, A², A³, A⁴, A⁵, A⁶, A⁷ and A⁸ independently of one another are identical or different and are a straight-chain or branched alkyl or alkenyl having 1 to 8 carbon atoms or cycloalkyl having 5 to 6 carbon atoms.

12. The process as claimed in one or more of claims 1 to 11, wherein a compound of the formula (II) is employed in which A¹, A², A³, A⁴, A⁵, A⁶, A⁷ and A⁸ independently of one another are identical or different and are a straight-chain or branched alkyl or alkenyl having 1 to 4 carbon atoms.

13. The process as claimed in one or more of claims 1 to 12, wherein a compound of the formula (II) is employed in which A¹A² = A³A⁴ or A¹A² = A³A⁴ = A⁵A⁶ or A¹A² =A³A⁴ = A⁵A⁶ = A⁷A⁸.

14. The process as claimed in one or more of claims 1 to 13, wherein a compound of the formula (II) is employed in which A¹ = A² = A³ = A⁴ or A¹ = A² = A³ = A⁴ = A⁵ = A⁶ or A¹ = A² = A³ = A⁴ = A⁵ = A⁶ = A⁷ = A⁸.

15. The process as claimed in one or more of claims 1 to 14, wherein a compound of the formula (II) is employed in which A¹A² or A¹A² and A³A⁴ or A¹A² and A³A⁴ and A⁵A⁶ or A¹A² and A³A⁴ and A⁵A⁶ and A⁷A⁸ are joined to one another directly or by way of O or N-A⁹ to form a saturated or unsaturated ring with 5 or 6 ring members.

16. The process as claimed in one or more of claims 1 to 6 and 15, wherein a compound of the formula (II) is employed in which A¹A² or A¹A² and A³A⁴ or A¹A² and A³A⁴ and A⁵A⁶ or A¹A² and A³A⁴ and A⁵A⁶ and A⁷A⁸ are joined to form a ring whose ring members comprise the N atom on which the respective radicals A¹ to A⁸ are located, if appropriate O or N-A⁹, and CH₂ groups.

17. The process as claimed in one or more of claims 1 to 16, wherein a compound of the formula (II) is employed in which B⁻ is F⁻, Cl⁻, Br⁻, HF₂⁻, I⁻, BF₄⁻, C₆H₅SO₃⁻, p-CH₃-C₆H₄-SO₃⁻, HSO₄⁻, PF₆⁻ or CF₃SO₃⁻.

18. The process as claimed in one or more of claims 1 to 17, wherein a compound of the formula (II) is employed in which B⁻ is F⁻, Cl⁻, Br⁻, HF₂⁻ or BF₄⁻.

19. The process as claimed in one or more of claims 1 to 18, wherein the compound of the formula (II) is employed in an amount of from 0.5 to 35% by weight, based on the compound which contains fluorine-exchangeable halogen.

20. The process as claimed in one or more of claims 1 to 19, wherein the solvent employed is a dipolar-aprotic, an aprotic or a protic solvent.

21. The process as claimed in one or more of claims 1 to 20, wherein the dipolar-aprotic solvent employed is dimethyl sulfoxide, dimethyl sulfone, sulfolane, dimethylformamide, dimethylacetamide, 1,3-dimethylimidazolin-2-one, N-methylpyrrolidone, hexamethylphos-phoramide, acetonitrile or benzonitrile or a mixture of these solvents.

22. The process as claimed in one or more of claims 1 to 20, wherein the aprotic solvent employed is an aromatic hydrocarbon, a chlorinated aromatic hydrocarbon or a mixture of these solvents.

23. The process as claimed in one or more of claims 1 to 20 and 22, wherein the aprotic solvent employed is benzene, toluene, ortho-, meta-, para-xylene, industrial mixtures of isomeric xylenes, ethylbenzene, mesitylene, ortho-, meta-, para-chlorotoluene, chlorobenzene, ortho-, meta-, para-dichlorobenzene or a mixture of these solvents.

24. The process as claimed in one or more of claims 1 to 20, wherein the protic solvent employed is methanol, ethanol, propanol, isopropanol, butanol, isobutanol, polyalkylene glycols having ethylene, propylene orbutylene units, or a mixture of these solvents.

25. The process as claimed in one or more of claims 1 to 24, wherein the reaction is conducted at from 60 to 250°C.

26. The process as claimed in one or more of claims 1 to 25, wherein the reaction is conducted at from 90 to 220°C.

27. The process as claimed in one or more of claims 1 to 26, wherein the reaction is conducted at from 120 to 200°C.

## Revendications

1. Procédé de préparation de composés fluorés, par réaction d'un composé contenant un halogène pouvant être remplacé par le fluor, avec un fluorure ou un mélange de fluorures de formule générale I
MeF (I)
dans laquelle Me représente un ion d'un métal alcalinoterreux, un ion NH₄⁺ ou un ion d'un métal alcalin, en présence ou en l'absence d'un solvant, à une température de 40 à 260°C, **caractérisé en ce qu'**on met en oeuvre la réaction en présence d'un composé ou d'un mélange de composés de formule générale (II) 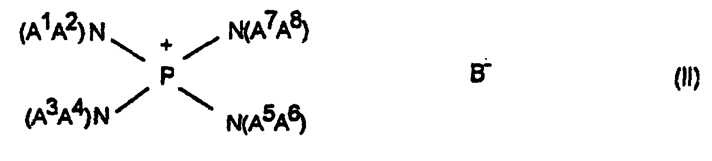
dans laquelle A¹, A², A³, A⁴, A⁵, A⁶, A⁷, A⁸ sont, indépendamment les uns des autres, identiques ou différents, et représentent chacun un groupe alkyle ou alcényle à chaîne droite ou ramifiée ayant de 1 à 12 atomes de carbone, cycloalkyle ayant de 4 à 8 atomes de carbone, aryle ayant de 6 à 12 atomes de carbone, aralkyle ayant de 7 à 12 atomes de carbone, ou encore A¹A², A³A⁴, A⁵A⁶, A⁷A⁸, indépendamment les uns des autres, sont identiques ou différents et sont reliés, directement ou par l'intermédiaire de O ou de N-A⁹, l'un à l'autre pour former un noyau ayant de 3 à 7 chaînons, A⁹ est un groupe alkyle ayant de 1 à 4 atomes de carbone, et B⁻ est un résidu d'acide monovalent ou l'équivalent d'un résidu d'acide polyvalent.

2. Procédé selon la revendication 1, **caractérisé en ce qu'**on utilise en tant que composé contenant un halogène pouvant être remplacé par le fluor un composé aromatique, ayant de 0 à 3 atomes d'azote dans le noyau, portant sur le noyau un substituant chloro ou bromo pouvant être remplacé par le fluor, composé qui contient éventuel-lement au moins un autre substituant, qui favorise la substitution nucléophile des composés aromatiques.

3. Procédé selon la revendication 1 ou 2, **caractérisé en ce qu'**on utilise un composé aromatique, comportant sur le noyau un substituant chloro ou bromo pouvant être remplacé par le fluor, ce composé comportant au moins un autre substituant de la série F, Cl, Br, I, NO₂, CF₃, CN, CHO, COF, COCl, SO₂F, SO₂Cl, OCF₃, SCF₃, SOCF₃, SO₂CF₃, COOR, COONRR', SO₂R, COR ou OR, ou encore un radical -CO-O-CO-, -CO-NR-CO- qui relie deux positions ortho, où R et R' sont, indépendamment l'un de l'autre, identiques ou différents, et représentent chacun un atome d'hydrogène, un groupe alkyle à chaîne droite ou ramifiée ayant de 1 à 6 atomes de carbone, un groupe aryle ayant de 6 à 12 atomes de carbone ou aralkyle ayant de 7 à 12 atomes de carbone, les groupes alkyle et aralkyle étant éventuellement mono- à trihalogénés.

4. Procédé selon l'une ou plusieurs des revendications 1 à 3, **caractérisé en ce qu'**on utilise un composé aromatique, comportant sur le noyau un substituant chloro ou bromo pouvant être remplacé par le fluor, composé qui possède au moins en tant qu'autre substituant un chlore ou un brome pouvant être remplacé par un fluor, et éventuellement au moins un autre substituant de la série F, NO₂, CF₃, CN, CHO, COF, COCl, SO₂F, SO₂Cl, OCF₃, SCF₃, SO₂CF₃, COOR, COONRR', SO₂R, COR, OR, -CO-O-CO- ou -CO-NR-CO-.

5. Procédé selon l'une ou plusieurs des revendications 1 à 4, **caractérisé en ce qu'**on utilise un composé de formule générale (III) 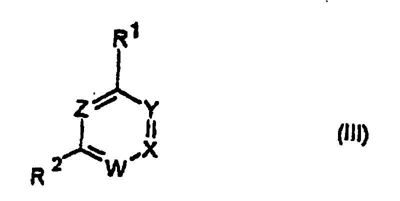 dans laquelle W est N ou C-R³, X est N ou C-R⁴, Y est N ou C-R⁵, Z est N ou C-R⁶ ; W, X et Y ne représentent pas simultanément N ; R¹, R², R³, R⁴, R⁵, R⁶ sont identiques ou différents et représentent chacun H, F, Cl, Br, I, NO₂, CF₃, CN, CHO, COF, COCl, SO₂F, SO₂Cl, OCF₃, SCF₃, SO₂CF₃, COOR, COONRR', SO₂R, COR, OR, un radical -CO-O-CO-, -CO-NR-CO- ou -CR"=CR"-CR"=CR"-, qui relie deux positions ortho ; R et R' ont les significations données ci-dessus ; et les radicaux R" sont, indépendamment les uns des autres, identiques ou différents et ont les mêmes significations que R¹ à R⁶ ; et au moins l'un des radicaux R¹ à R⁶ est le chlore ou le brome.

6. Procédé selon l'une ou plusieurs des revendications 1 à 5, **caractérisé en ce qu'**on utilise un composé de formule (III) dans laquelle un seul des radicaux R¹ à R⁶ est le chlore ou le brome ; aucun des radicaux W, X, Y, Z ne représente un atome d'azote ; et au moins l'un des radicaux restants parmi les radicaux R¹ à R⁶ est NO₂, CF₃, CN, CHO, COF, COCl, SO₂F, SO₂Cl, OCF₃, SCF₃, SO₂CF₃, COOR, COONRR', SO₂R, COR, OR, -CO-O-CO-, -CO-NR-CO- ou -CR"=CR"-CR"=CR"-.

7. Procédé selon l'une ou plusieurs des revendications 1 à 5, **caractérisé en ce qu'**on utilise un composé de formule (III) dans laquelle au moins deux des radicaux R¹ à R⁶ est le chlore ou le brome ; les radicaux W, X, Y, Z représentent de 0 à 3 atomes d'azote ; et les autres radicaux de l'ensemble R¹ à R⁶ peuvent tous être des atomes d'hydrogène.

8. Procédé selon l'une ou plusieurs des revendications 1 à 7, **caractérisé en ce qu'**on utilise en tant que fluorure de formule (I) le fluorure de lithium, le fluorure de sodium, le fluorure de potassium, le fluorure de rubidium, le fluorure de césium ou un mélange de ceux-ci.

9. Procédé selon l'une ou plusieurs des revendications 1 à 8, **caractérisé en ce qu'**on utilise le fluorure de formule (II) selon une proportion, avec l'équivalent de l'halogène devant être remplacé, de (0,5 à 10):(1), en particulier de (0,8 à 5) : (1) , de préférence de (1 à 2) : (1) .

10. Procédé selon l'une ou plusieurs des revendications 1 à 9, **caractérisé en ce qu'**on utilise un composé de formule (II) dans laquelle A¹, A², A³, A⁴, A⁵, A⁶, A⁷, A⁸ sont, indépendamment les uns des autres, identiques ou différents, et représentent chacun un groupe alkyle ou alcényle à chaîne droite ou ramifiée ayant de 1 à 12 atomes de carbone, ou cycloalkyle ayant de 4 à 8 atomes de carbone.

11. Procédé selon l'une ou plusieurs des revendications 1 à 10, **caractérisé en ce qu'**on utilise un composé de formule (II) dans laquelle A¹, A², A³, A⁴, A⁵, A⁶, A⁷, A⁸ sont, indépendamment les uns des autres, identiques ou différents, et représentent chacun un groupe alkyle ou alcényle à chaîne droite ou ramifiée ayant de 1 à 8 atomes de carbone, ou cycloalkyle ayant de 5 à 6 atomes de carbone.

12. Procédé selon l'une ou plusieurs des revendications 1 à 11, **caractérisé en ce qu'**on utilise un composé de formule (II) dans laquelle A¹, A², A³, A⁴, A⁵, A⁶, A⁷, A⁸ sont, indépendamment les uns des autres, identiques ou différents, et représentent chacun un groupe alkyle ou alcényle à chaîne droite ou ramifiée ayant de 1 à 4 atomes de carbone.

13. Procédé selon l'une ou plusieurs des revendications 1 à 12, **caractérisé en ce qu'**on utilise un composé de formule (II) dans laquelle A¹A² = A³A⁴, ou A¹A² = A³A⁴ = A⁵A⁶, ou A¹A² = A³A⁴ = A⁵A⁶ = A⁷A⁸.

14. Procédé selon l'une ou plusieurs des revendications 1 à 13, **caractérisé en ce qu'**on utilise un composé de formule (II) dans laquelle A¹ = A² = A³ = A⁴, ou A¹ = A² = A³ = A⁴ = A⁵ = A⁶, ou A¹ = A² = A³ = A⁴ = A⁵ = A⁶ = A⁷ = A⁸.

15. Procédé selon l'une ou plusieurs des revendications 1 à 14, **caractérisé en ce qu'**on utilise un composé de formule (II) dans laquelle A¹A² ou A¹A² et A³A⁴ ou A¹A² et A³A⁴ et A⁵A⁶ ou A¹A² et A³A⁴ et A⁵A⁶ et A⁷A⁸ sont reliés l'un à l'autre, directement ou par l'intermédiaire de O ou de N-A⁹, pour donner un noyau saturé ou insaturé ayant 5 ou 6 chaînons.

16. Procédé selon l'une ou plusieurs des revendications 1 à 6 et 15, **caractérisé en ce qu'**on utilise un composé de formule (II) dans laquelle A¹A² ou A¹A² et A³A⁴ ou A¹A² et A³A⁴ et A⁵A⁶ ou A¹A² et A³A⁴ et A⁵A⁶ et A⁷A⁸ sont reliés pour former un noyau qui en tant que chaînons comporte l'atome d'azote auquel sont reliés les différents radicaux A¹ à A⁸, éventuellement O ou N-A⁹, et des groupes CH2.

17. Procédé selon l'une ou plusieurs des revendications 1 à 16, **caractérisé en ce qu'**on utilise un composé de formule (II) dans laquelle B⁻ est F⁻, Cl⁻, Br⁻, HF₂⁻, I⁻, BF₄⁻, C₆H₅SO₃⁻, p-CH₃-C₆H₄-SO₃⁻, HSO₄⁻, PF₆⁻, CF₃SO₃⁻.

18. Procédé selon l'une ou plusieurs des revendications 1 à 17, **caractérisé en ce qu'**on utilise un composé de formule (II) dans laquelle B⁻ est F⁻, Cl⁻, Br⁻, HF₂⁻, BF₄⁻.

19. Procédé selon l'une ou plusieurs des revendications 1 à 18, **caractérisé en ce qu'**on utilise le composé de formule (II) en une quantité de 0,5 à 35 % en poids par rapport au composé qui contient un halogène pouvant être remplacé par le fluor.

20. Procédé selon l'une ou plusieurs des revendications 1 à 19, **caractérisé en ce qu'**on utilise en tant que solvant un solvant aprotique dipolaire, un solvant aprotique ou un solvant protique.

21. Procédé selon l'une ou plusieurs des revendications 1 à 20, **caractérisé en ce qu'**on utilise en tant que solvant aprotique dipolaire le diméthylsulfoxyde, la diméthylsulfone, le sulfolanne, le diméthylformamide, le diméthylacétamide, la 1,3-diméthylimidazoline-2-one, la N-méthylpyrrolidone, l'hexaméthylphosphorotriamide, l'acétonitrile, le benzonitrile ou un mélange de ces solvants.

22. Procédé selon l'une ou plusieurs des revendications 1 à 20, **caractérisé en ce qu'**on utilise en tant que solvant aprotique un hydrocarbure aromatique, un hydrocarbure aromatique chloré ou un mélange de ces solvants.

23. Procédé selon l'une ou plusieurs des revendications 1 à 20 et 22, **caractérisé en ce qu'**on utilise en tant que solvant aprotique le benzène, le toluène, l'ortho-, le méta-, le paraxylène, des mélanges techniques de xylènes isomères, l'éthylbenzène, le mésitylène, l'ortho-, le méta-, le parachlorotoluène, le chlorobenzène, l'ortho-, le méta-, le paradichlorobenzène, ou un mélange de ces solvants.

24. Procédé selon l'une ou plusieurs des revendications 1 à 20, **caractérisé en ce qu'**on utilise en tant que solvant protique le méthanol, l'éthanol, le propanol, l'isopropanol, le butanol, l'isobutanol, des polyalkylèneglycols comportant des motifs éthylène, propylène ou butylène, ou un mélange de ces solvants.

25. Procédé selon l'une ou plusieurs des revendications 1 à 24, **caractérisé en ce qu'**on met en oeuvre la réaction à une température de 60 à 250°C.

26. Procédé selon l'une ou plusieurs des revendications 1 à 25, **caractérisé en ce qu'**on met en oeuvre la réaction à une température de 90 à 220°C.

27. Procédé selon l'une ou plusieurs des revendications 1 à 26, **caractérisé en ce qu'**on met en oeuvre la réaction à une température de 120 à 200°C.
